# FASCICULE DE BREVET EUROPEEN

(11) **EP 1 379 547 B1**
(45) Date de publication et mention de la délivrance du brevet: **21.12.2005**
(21) Numéro de dépôt: 02713992.2
(22) Date de dépôt: 22.04.2002
(51) Int. Cl.: C07K 7/64, A61K 38/13, A61K 47/48

(54) **CYCLOSPORINE MODIFIEE UTILISABLE EN TANT QUE PRO-DROGUE ET SON UTILISATION**
MODIFIZIERTES, ALS MEDIKAMENTVORSTUFE ANWENDBARES CYCLOSPORIN UND DESSEN VERWENDUNG
MODIFIED CYCLOSPORIN AS A PRO-DRUG AND USE THEREOF AS A MEDICAMENT

(30) Priorité: 20.04.2001 CH 72001
(43) Date de publication de la demande: 14.01.2004
(73) Titulaire: DEBIOPHARM S.A., 1002 Lausanne (CH)
(72) Inventeur: WENGER, Roland, CH-4125 Riehen (CH); MUTTER, Manfred, CH-1092 Belmont-sur-Lausanne (CH); HAMEL, Arnaud, CH-1004 Lausanne (CH); HUBLER, Francis, CH-4310 Rheinfelden (CH)
(74) Mandataire: Besse, François
(86) Numéro de dépôt international: PCT/CH2002/000222
(87) Numéro de publication internationale: WO 2002/085928

(56) Documents cités:
- WO-A-00/08033
- WO-A-00/67801
- WO-A-01/05818
- WO-A-01/05819

## Description

La présente invention concerne une pro-drogue constituée d'un undéca-peptide cyclique et son utilisation en tant que médicament, destiné, en particulier, au traitement de pathologies oculaires.

Les cyclosporines constituent une classe structurellement distincte de peptides cycliques qui ont en commun d'être constitués d'un enchaînement de onze acides aminés, certains étant atypiques, soit du fait de leur configuration D, soit du fait de la structure chimique élaborée de leur chaîne latérale, soit encore par le fait de l'alkylation du groupe amine.

A ce jour, une trentaine de cyclosporines ont été isolées à partir de source fongique et de nombreux undéca-peptides cycliques analogues à ces produits naturels ont été obtenus par hémisynthèse ou par synthèse totale. Parmi ces analogues de undéca-peptides cycliques, on trouve également des peptolides ou depsipeptides, c'est-à-dire des polypeptides cycliques contenant également dans leur enchaînement des liens ester.

Dans la suite de cette description et à moins qu'il n'en soit précisé autrement, par "cyclosporine", on entendra aussi bien les undéca-peptides cycliques obtenus de source naturelle que leurs analogues obtenus par hémisynthèse ou par synthèse totale, y compris les peptolides obtenus de source naturelle ou leurs analogues obtenus par hémisynthèse ou par synthèse totale.

Le premier membre de cette famille des cyclosporines à avoir été isolé puis identifié a été la Cyclosporine A. L'enchaînement peptidique constituant son cycle undéca-peptidique est le suivant:

-MeBmt-Abu-Sar-MeLeu-Val-MeLeu-Ala-(D)Ala-MeLeu-MeLeu-MeVal-

sa structure chimique développée étant la suivante:

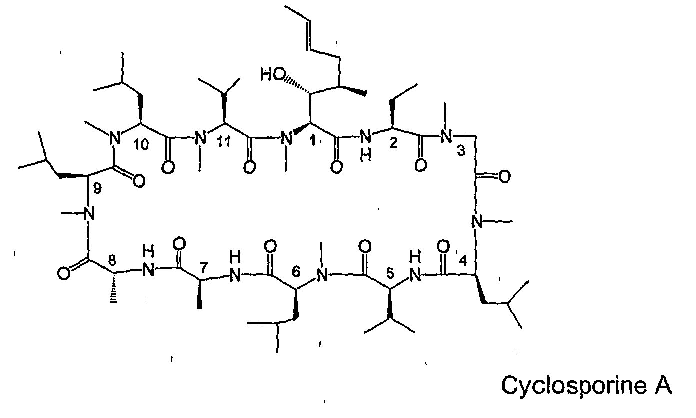

Parmi les acides aminés atypiques que comprend cet undéca-peptide cyclique, on note en particulier celui qui se trouve en position 1, à savoir la N-méthyl-(4R)-4-((E)-2-butényl)-4-méthyl-L-thréonine, noté MeBmt.

Cet acide aminé est spécifique aux cyclosporines, le groupe éthylénique pouvant éventuellement être réduit. Il possède un groupe amine qui est méthylé. En outre, le groupe hydroxyle qu'il porte est très remarquable en ce sens qu'il est le seul groupe de tout cet undéca-peptide cyclique susceptible de conduire à des modifications chimiques. On peut en outre constater d'ores et déjà qu'il se trouve dans un environnement stérique fortement encombré rendant toute approche de réactif extrêmement délicate.

Ces undéca-peptides cycliques, qu'ils soient d'origine naturelle ou obtenus par synthèse, présentent un large spectre d'activités biologiques parmi lesquelles les plus connues sont les activités immunosuppressives, anti-inflammatoires, anti-parasitaires, ou des activités permettant de contrecarrer ou de diminuer la résistance de tumeurs cancéreuses à d'autres médications. Certains de ces undéca-peptides cycliques se sont avérés posséder des activités anti-virales prometteuses, en particulier, dans le traitement du SIDA en inhibant la réplication du virus de l'immunodéficience humaine de type 1 (VIH-1).

A ce titre, un certain nombre d'undéca-peptides cycliques, obtenus par hémisynthèse et ayant une structure analogue à celle de la Cyclosporine A, mais dont la nature des acides aminés se trouvant en position 4, ou en position 3 et 4, par rapport à l'acide aminé MeBmt a été modifiée, ont été décrits dans la demande de brevet WO 00/01715 déposée par la présente demanderesse.

De récents développements pharmacologiques ont permis d'espérer que l'effet immuno-modérateur des cyclosporines, en particulier celui de la Cyclosporine A, effet qui est réversible, non myélotoxique et pour lequel peu d'effets secondaires ont été répertoriés, puisse être mis à profit en particulier dans le domaine ophtalmique, pour un traitement local, en particulier, de pathologies superficielles de l'oeil et de ses annexes.

Parmi ces pathologies, on trouve, entre autres, les kératoconjonctivites sèches, appelées également syndrome de l'oeil sec, le syndrome de Sjögren, les kératoconjonctivites allergiques, en particulier, celles résistantes aux corticostéroïdes, les conjonctivites muco-synéchiantes, les kératites stromales herpétiques, les kératites limbiques immunitaires, la kératite de Thygeson, la prévention du rejet des greffes de cornée, et comme traitement adjuvant d'une chirurgie filtrante.

Les undéca-peptides cycliques de la famille des cyclosporines possèdent un caractère fortement hydrophobe réduisant d'autant leur solubilité dans l'eau. Ce caractère est lié à la nature de la chaîne latérale de la plupart de leurs acides aminés, mais également au fait que certain de ces acides aminés ont leur groupe amine méthylé, limitant ainsi le nombre possible de formation de liaison hydrogène intermoléculaire entre le undéca-peptide cyclique et, par exemple, un milieu de solubilisation aqueux.

De ce fait, l'administration par voie intra-veineuse (i.v.) de ces cyclosporines nécessite des mises au point de formulations galéniques très complexes, principalement sous la forme d'émulsions, parfois de stabilité précaire et de manipulations délicates, et sources d'effets secondaires délétères.

A titre d'exemple, l'une des préparations pour perfusion i.v. de Cyclosporine A, disponible dans le commerce sous la marque de commerce Sandimmun, est constituée d'une micro-émulsion utilisant comme excipient une huile de ricin polyoxyéthylénée connue sous le nom de marque de commerce Crémophor. Cette préparation est conservée sous la forme d'un concentré et doit être diluée juste avant son administration.

Du fait de l'emploi de cette huile de ricin qui est connue pour mettre en solution certain des composants de matières synthétiques, le fabricant recommande de n'utiliser lors de la manipulation de cette préparation que du matériel en verre ou, à défaut, en un matériau synthétique conforme aux "normes de la Pharmacopée Européenne pour les récipients destinés à contenir du sang", tous ces matériaux devant être exempts d'huile de silicone et de matière grasses.

De plus, il met en garde le clinicien du fait que cette huile de ricin est susceptible d'entraîner des réactions anaphylactoïdes et, de ce fait, recommande que l'administration par voie intraveineuse soit réservée aux cas où l'administration orale est impossible.

Le développement des applications prometteuses des cyclosporines par administration locale en ophtalmologie reste ralenti du fait également des difficultés à mettre au point des formulations galéniques appropriées, présentant en particulier une bonne tolérance locale et ne troublant pas la vision par la présence d'agents visqueux.

Ainsi, et à titre d'exemple, Robert et al. ont récemment passé en revue, dans *J. Fr. Ophtalmol.,* 2001, 24(5), 527, toutes les difficultés techniques qu'il y a à mettre au point, du fait du caractère lipophile, des formulations galéniques permettant d'administrer la Cyclosporine A par voie locale en ophtalmologie et tous les problèmes de tolérance locale qu'entraînent ces formulations.

L'une des conclusions que l'on peut tirer de cette revue est que, à ce jour, il n'existe aucune formulation sous forme d'un collyre qui soit apte à être administrée localement pour le traitement d'affections de l'oeil et de ces annexes. Cette conclusion peut être élargie pour l'usage de la cyclosporine pour le traitement local d'affections des muqueuses ou d'affections cutanées.

Il existe par conséquent toujours un besoin de mettre à disposition du clinicien des cyclosporines, qu'elles soient d'origine naturelle ou synthétique, ou des dérivés de ces cyclosporines qui puissent être rendues facilement administrables à un patient, en particulier par voie locale ou intra-veineuse, ceci en évitant l'emploi de formulations galéniques complexes, à stabilité précaire et à manipulation délicate, et sources d'effets secondaires délétères.

Ce besoin existe d'autant plus si ces cyclosporines, d'origines naturelle ou synthétique, doivent être appliquées localement au niveau de l'oeil ou de ses annexes.

L'une des possibilités qui s'offre au spécialiste lorsque qu'il est confronté au problème de rendre assimilable dans un milieu physiologique une molécule pharmacologiquement active hydrophobe est de la modifier chimiquement afin de lui conférer un caractère hydrophile.

Afin d'éviter de d'altérer les propriétés pharmacologiques d'une telle molécule pharmacologiquement active, cette modification chimique peut consister à préparer un précurseur si possible inactif, de cette molécule pharmacologiquement active qui, une fois administré et sous l'effet des conditions physiologiques régnantes localement au sein de l'organisme, sera modifié chimiquement ou enzymatiquement de telle sorte que la molécule pharmacologiquement active soit libérée, si possible, soit sur le site où son action pharmacologique doit se produire, soit dans le sang qui transportera cette molécule pharmacologiquement active ainsi libérée sur son site d'action, ceci correspondant au concept de "pro-drogue". Dans la suite de cette description, le précurseur en question de ladite molécule pharmacologiquement active est appelé "pro-drogue".

Il est déjà connu de modifier chimiquement la structure de la Cyclosporine A en vue de conférer au produit obtenu un caractère hydrophile.

Ainsi, Rothbard et al. ont décrit dans la demande de brevet WO 01/13957 une méthode pour améliorer l'administration de molécules pharmacologiquement actives et pour leur permettre de traverser le derme et les membranes épithéliales consistant à greffer pur ces molécules, de façon réversible, une chaîne latérale constituée de fragments d'une chaîne poly-arginine. Parmi ces molécules pharmacologiquement actives, on trouve des molécules à caractère hydrophobe comme la Cyclosporine A.

Toutefois, de tels conjugués sont de manipulation et de conservation extrêmement délicates du fait, comme il est indiqué dans cette demande citée, que la molécule pharmacologiquement active se trouve libérée dès que le pH du milieu dépasse 7. De plus, lors de la libération de cette molécule pharmacologiquement active, les fragments de chaîne poly-arginine se trouvent relargués au sein de l'organisme. Ces poly-arginines, étant connues pour leur toxicité et leur pouvoir irritant, provoqueraient des irritations telles qu'un usage dans le domaine ophtalmique de tels conjugués de cyclosporine n'est pas envisageable.

Crooks et al. décrivent dans la demande de brevet WO 00/67801 la préparation de pro-drogues d'agents anti-inflammatoires tels que le flurbiprofen afin de pouvoir les administrer localement au contact de l'oeil, tout en évitant cette fois toute irritation locale. Ils y sont parvenus, pour un certain nombre de médicaments, en introduisant des chaînes oxygénées ou poly-oxygénées.

En revanche, en voulant faire subir à la Cyclosporine A des mêmes modifications chimiques, ils ne sont parvenus à obtenir que des produits qu'ils décrivent comme étant stables, autrement dit qui ne se clivent pas pour libérer la Cyclosporine A, que se soit au contact du sérum humain ou dans un tampon phosphate se trouvant à pH 7,4. D'autres pro drogues de cyclosporines sont décrites dans WO-A-2001 05819 (Kuhnil) et WO-A-2000 08333 (University of Kansas).

La présente invention a par conséquent pour but de mettre à la disposition du clinicien des pro-drogues de undéca-peptides cycliques de la famille des cyclosporines, d'une part, qui puissent être administrables au sein du milieu physiologique sans avoir à mettre au point des formulations galéniques élaborées, d'autre part, qui puissent être conservés, puis manipulée et administrée sans avoir à se préoccuper en particulier des conditions de pH du milieu environnant.

La présente invention a également pour but de mettre à la disposition du clinicien des pro-drogues de undéca-peptides cycliques de la famille des cyclosporines qui puissent être administrées localement d'une façon générale, et en particulier sur la surface oculaire ou sur les muqueuses, et qui puissent ensuite libérer dans un temps de demi-vie approprié le undéca-peptide cyclique pharmacologiquement actif et ceci sans irritation locale.

A cet effet, la présente invention concerne une pro-drogue constituée d'un undéca-peptide cyclique dont l'enchaînement peptidique comprend au moins un résidu d'acide aminé de formule générale (I) suivante: 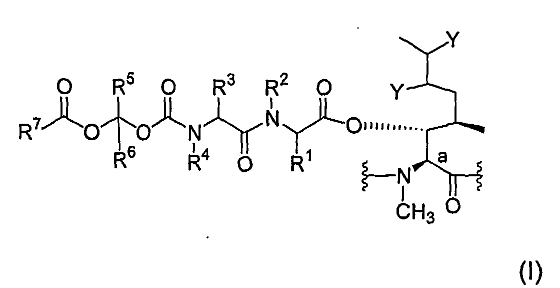 dans laquelle:
- l'atome de carbone C^{a} constitue l'un des maillons du cycle undéca-peptidique;
- les substituants Y représentent chacun un atome d'hydrogène ou constituent ensemble une liaison;
- les substituants R¹ et R³ représentent, indépendamment l'un de l'autre, un atome d'hydrogène, un groupe aralkyle, un groupe alkaryle, un groupe hétéroalkyle, un groupe hétérocyclique, un groupe alkyl-hétérocyclique, un groupe hétérocyclique-alkyle ou un groupe alkyle ayant de 1 à 6 atomes de carbone, linéaire ou ramifié, lesdits groupes étant éventuellement substitués par au moins un des groupements choisis parmi -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH)₂, et les différentes formes salifiées de ces groupements, chacun des substituants R⁸ représentant indépendamment l'un de l'autre un groupe alkyle, linéaire ou ramifié, ayant de 1 à 6 atomes de carbone;
- les substituants R² et R⁴ représentent, indépendamment l'un de l'autre, un atome d'hydrogène, un groupe alkaryle, ou un groupe alkyle ayant de 1 à 6 atomes de carbone, linéaire ou ramifié;
- les substituants R⁵ et R⁶ représentent, indépendamment l'un de l'autre, un atome d'hydrogène, un groupe aralkyle, un groupe alkyle ayant de 1 à 6 atomes de carbone, linéaire ou ramifié; et
- le substituant R⁷ représente un groupe aralkyle, un groupe alkaryle, un groupe hétéroalkyle, un groupe hétérocyclique, un groupe alkyl-hétérocyclique, un groupe hétérocyclique-alkyle ou un groupe alkyle ayant de 1 à 6 atomes de carbone, linéaire ou ramifié, lesdits groupes étant éventuellement substitués par au moins un des groupements choisis parmi -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH)₂, et les différentes formes salifiées de ces groupements, chacun des substituants R⁸ ayant la définition précédente.

Lorsque les deux substituants Y constituent ensemble une liaison, ledit résidu d'acide aminé de formule générale (I) dérive d'un résidu de la N-méthyl-(4R)-4-((E)-2-butényl)-4-méthyl-L-thréonine dans lequel le groupe hydroxyle de la thréonine a été estérifié de façon appropriée et la molécule pharmacologiquement active qui sera libérée lorsque la pro-drogue sera clivée au sein de l'organisme sera constituée d'un undéca-peptide cyclique dont l'enchaînement peptidique comprend au moins un résidu N-méthyl-(4R)-4-((E)-2-butényl)-4-méthyl-L-thréonine (MeBmt).

De même, lorsque les deux substituants Y représentent chacun un atome d'hydrogène, ledit résidu d'acide aminé de formule générale (I) dérive d'un résidu de la N-méthyl-(4R)-4-butyl-4-méthyl-L-thréonine dans lequel le groupe hydroxyle de la thréonine a été estérifié de façon appropriée et la molécule pharmacologiquement active qui sera libérée lorsque la pro-drogue sera clivée au sein de l'organisme sera constitué d'un undéca-peptide cyclique dont l'enchaînement peptidique comprend au moins un résidu N-méthyl-(4R)-4-butyl)-4-méthyl-L-thréonine (Dh-MeBmt).

De préférence, dans la formule générale (I) définissant ledit résidu d'acide aminé, au moins l'un des substituants R¹ et R³ représente un groupe aralkyle, un groupe alkaryle, un groupe hétéroalkyle, un groupe hétérocyclique, un groupe alkyl-hétérocyclique, un groupe hétérocyclique-alkyle ou un groupe alkyle ayant de 1 à 6 atomes de carbone, linéaire ou ramifié, chacun desdits groupes étant substitué par au moins un des groupements choisis parmi -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH)₂, et les différentes formes salifiées de ces groupements, chacun des substituants R⁸ ayant la définition précédente. Ces groupements, reconnus pour leur caractère polaire, améliorent grandement le caractère hydrophile conféré à ladite pro-drogue.

De préférence encore, lesdits groupes aralkyle, alkaryle, hétéroalkyle, hétérocyclique, alkyl-hétérocyclique, hétérocyclique-alkyle ou alkyle précédents sont substitués par au moins un des groupements choisis parmi -NR⁸₂, -N⁺R⁸₃, -OPO(OH)₂ ou les différentes formes salifiées de ces groupes, chacun des substituants R⁸ ayant la définition, précédente.

De préférence encore, au moins l'un desdits substituants R¹ et R³ représentent un groupe alkyle linéaire ayant de 1 à 6 atomes de carbone substitués par au moins un des groupements choisis parmi -NR⁸₂, -N⁺R⁸₃, -OPO(OH)₂ ou les différentes formes salifiées de ces groupements, chacun des substituants R⁸ ayant la définition précédente.

Lorsque lesdits substituants R¹ et R³ représentent un groupe alkyle linéaire ayant de 1 à 6 atomes de carbone substitués par au moins un des groupements choisis parmi -NR⁸₂, -N⁺R⁸₃, -OPO(OH)₂ ou ses différentes formes salifiées de ces groupements, chacun des substituants R⁸ ayant la définition précédente, les résidus d'acides aminés correspondants dérivent de préférence:
- soit de résidus de sérine, de homo-sérine, de thréonine, de allo-thréonine, de N-méthyl-sérine, de N-méthyl-thréonine, de N-méthyl-homo-sérine, sous l'une quelconque des configurations (D) ou (L), de préférence (L), et dans lesquels le groupe hydroxyle a été fonctionnalisé de façon appropriée telle que la chaîne latérale des ces résidus d'acides aminés porte les groupements polaires et/ou solubilisants; ou
- soit de résidus de lysine, d'ornithine, d'arginine, de N-delta-méthyl-arginine, de N-alpha-méthyl-arginine, de N-méthyl-lysine, sous l'une quelconque des configurations (D) ou (L), de préférence (L), et dans lesquels le groupe respectivement amine ou imine a été fonctionnalisé de façon appropriée telle que la chaîne latérale des ces résidus d'acides aminés porte les groupements polaires et/ou solubilisants.

Lorsque les substituants R¹, R² et/ou R³, R⁴ formant les couples (R¹, R²) et/ou (R³, R⁴) sont des groupes alkyle ayant de 1 à 6 atomes de carbone, ils peuvent former au sein de chaque couple une chaîne alkylène formant avec l'atome de carbone et l'atome d'azote qui les portent un cycle. De préférence, ils constituent la chaîne latérale d'un résidu de proline.

Lorsque les substituants R¹ et R³ représentent, indépendamment l'un de l'autre, un atome d'hydrogène, un groupe aralkyle, un groupe alkaryle, un groupe hétéroalkyle, un groupe hétérocyclique, un groupe alkyl-hétérocyclique, un groupe hétérocyclique-alkyle ou un groupe alkyle ayant de 1 à 6 atomes de carbone, linéaire ou ramifié, mais que lesdits groupes ne sont pas substitués par au moins un des groupements choisis parmi -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH)₂, et les différentes formes salifiées de ces groupements, alors ils représentent de préférence les chaînes latérales de résidus d'acides aminés, sous des configurations (D) ou (L), de préférence (L), ou de résidus desdits acides aminés sous des formes protégées et/ou activées et ayant éventuellement leur groupe amine alkylé habituellement disponibles dans le commerce. De préférence encore, lesdits résidus d'acides aminés sont choisis parmi les vingt acides aminés habituellement appelés acides aminés naturels.

De préférence également, dans la formule générale (I), les substituants R⁵ et R⁶ ne peuvent représenter simultanément un atome d'hydrogène. De préférence également, au moins l'un desdits substituants R⁵ et R⁶ représente un groupe alkyle ayant de 1 à 6 atomes de carbone, linéaire ou ramifié, et le substituant R⁷ représente un groupe aralkyle ou un groupe alkyle ayant de 1 à 6 atomes de carbone, linéaire ou ramifié.

De préférence encore, lesdits substituants R⁵ et R⁶ représentent, indépendamment l'un de l'autre, un atome d'hydrogène ou un groupe méthyle.

De préférence, ladite pro-drogue est constituée d'un undéca-peptide cyclique dont l'enchaînement peptidique comprend un seul résidu d'acide aminé de formule générale (I) et forme ainsi un cycle undéca-peptidique avec une séquence linéaire de dix acides aminés de formule générale (II) suivante:

-T-U-V-W-MeLeu-Ala-X-MeLeu-Z-MeVal- (II)

dans laquelle:
- T est choisi parmi les acides aminés Ala, Abu, Nval, Val et Thr;
- U est choisi parmi les acides aminés Sar, (D)MeSer, (D)MeAla et (D)MeSer(OCOR⁹), avec R⁹ représentant un atome d'hydrogène, un groupe alkaryle, ou un groupe alkyle ayant de 1 à 6 atomes de carbone, linéaire ou ramifié;
- V représente un acide aminé de formule générale (N-R¹⁰)aa, aa étant choisi parmi les acides aminés Val, Leu, Ile, Thr, Phe, Tyr, Thr et R¹⁰ étant un groupe akyle linéaire ou ramifié ayant de 1 à 6 atomes de carbone;
- W est choisi parmi les acides aminés Val, NVal et Leu;
- X est choisi parmi les acides aminés (D)Ala, (D)Ser, (D)Hiv, (D)Val et (D)Thr, avec (D)Hiv représentant un résidu de l'acide D-2-hydroxy-isovalérique; et
- Z est choisi parmi les acides aminés Leu et MeLeu.

Ainsi, lorsque, dans ledit résidu d'acide aminé de formule générale (I), les deux substituants Y représentent chacun un atome d'hydrogène, la molécule pharmacologiquement active qui sera libérée, au cours du clivage de la pro-drogue au sein de l'organisme, sera constitué d'un undéca-peptide cyclique de la famille des cyclosporines dont l'enchaînement peptidique contient un résidu N-méthyl-(4R)-4-butyl)-4-méthyl-L-thréonine (Dh-MeBmt).

De même, lorsque, dans ledit résidu d'acide aminé de formule générale (I), les deux substituants Y constituent ensemble une liaison, la molécule pharmacologiquement active qui sera libérée, au cours du clivage de la pro-drogue au sein de l'organisme sera constitué d'un undéca-peptide cyclique de la famille des cyclosporines dont l'enchaînement peptidique comprend un résidu N-méthyl-(4R)-4-((E)-2-butényl)-4-méthyl-L-thréonine (MeBmt).

De préférence, ces undéca-peptides cycliques correspondent aux cyclosporines déjà décrites dans la littérature pour posséder des propriétés pharmacologiques et ayant toutes dans leur enchaînement peptidique, soit un résidu N-méthyl-(4R)-4-((E)-2-butényl)-4-méthyl-L-thréonine (MeBmt), soit un résidu N-méthyl-(4R)-4-butyl)-4-méthyl-L-thréonine (Dh-MeBmt).

De préférence encore, la séquence linéaire des dix résidus d'acides aminés restants constituant, avec ledit résidu d'acide aminé de formule générale (I), lesdits undéca-peptides cycliques est choisie parmi les séquences suivantes de formules (III) à (XIV):

-Abu-Sar-MeLeu-Val-MeLeu-Ala-(D)Ala-MeLeu-MeLeu-MeVal- (III);

-Abu-(D)MeAla-EtVal-Val-MeLeu-Ala-(D)Ala-MeLeu-MeLeu-MeVal- (IV);

-Thr-Sar-MeLeu-Val-MeLeu-Ala-(D)Ala-MeLeu-MeLeu-MeVal- (V);

-Val-Sar-MeLeu-Val-MeLeu-Ala-(D)Ala-MeLeu-MeLeu-MeVal- (VI);

-NVal-Sar-MeLeu-Val-MeLeu-Ala-(D)Ala-MeLeu-MeLeu-MeVal- (VII);

-Val-(D)MeAla-MeLeu-Val-MeLeu-Ala-(D)Ala-MeLeu-MeLeu-MeVal- (VIII);

-Val-Sar-MeLeu-Val-MeLeu-Ala-(D)Val-MeLeu-Leu-MeVal- (IX);

-Val-Sar-MeLeu-Val-MeLeu-Ala-(D)Thr-MeLeu-Leu-MeVal- (X);

-Abu-(D)MeSer(OAc)-MeLeu-Val-MeLeu-Ala-(D)Ala-MeLeu-Leu-MeVal- (XI);

-Abu-Sar-MeLeu-Val-MeLeu-Ala-(D)Ser-MeLeu-MeLeu-MeVal- (XII);

-Thr-Sar-MeLeu-Leu-MeLeu-Ala-(D)Hiv-MeLeu-Leu-MeVal- (XIII);

et

-Abu-Sar-MeLeu-Val-MeLeu-Ala-(D)Val-MeLeu-Leu-MeVal- (XIV).

La molécule pharmacologiquement active qui sera libérée lors du clivage de la pro-drogue au sein de l'organisme sera alors respectivement l'une des cyclosporines suivantes avec, selon les cas, un résidu dérivé de la thréonine avec une chaîne butényle (MeBmt) ou butyle (Dh-MeBmt):
Cyclosporine A (CsA); (D)MeAla³EtVal⁴CsA (WO 00/01715); Cyclosporine C (CsC); Cyclosporine D (CsD); Cyclosporine G (CsG); (D)MeAla³CsD; (D)Val⁸Csl; (D)Thr⁸Csl; (D)MeSer(OAc)³CsT; (D)Ser³CsA (Progress in Medicinal Chemistry, Vol 25, ed Ellis and West, Elsevier Science Publ., Biomedical Division, 1998, pp 1-33); Thr²Leu⁵(D)Hiv⁸Leu¹⁰CsC (The Journal of Biological Chemistry, 1991, 266(24), 15570); (D)Val⁸Leu¹⁰CsA; les cyclosporines A, C, D, G, I et T étant décrites dans Progress in the Chemistry of Organic Natural Products, 1986, 50, 124, les cyclosporines restantes étant préparées par analogie au procédé décrit dans Helvetica Chimica Acta, 1984, 67,502.

De préférence encore, les pro-drogues de la présente invention ont respectivement les formules (XV) et (XVI) suivantes: 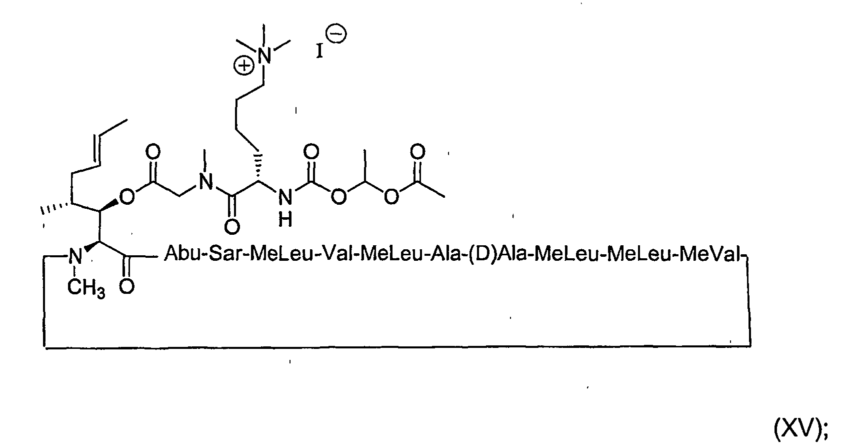 et

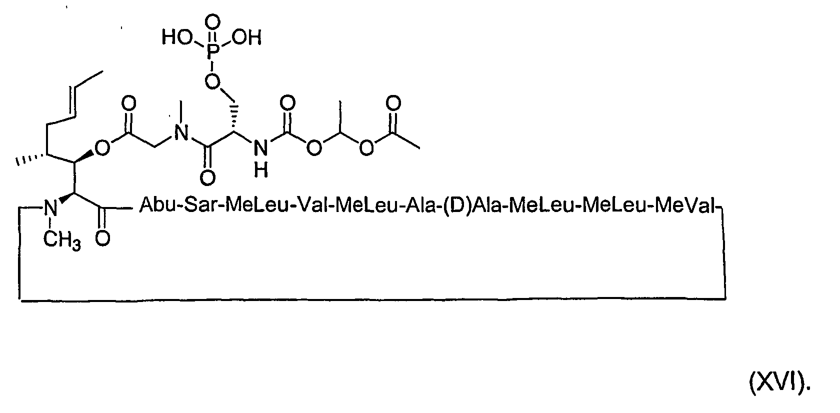

La pro-drogue selon la présente invention peut être préparée en appliquant des méthodes de synthèses chimiques bien connues du spécialiste de la chimie des peptides, et tout particulièrement de la chimie des cyclosporines.

Par le choix approprié des différents substituants définissant le résidu d'acide aminé de formule générale (I), les pro-drogues selon la présente invention se sont avérées posséder, de façon remarquable, un caractère hydrophile grandement amélioré par rapport à la molécule pharmacologiquement active générée lors du clivage de ladite pro-drogue. A titre d'exemple, la solubilité de certaines pro-drogues de la présente invention générant après clivage la Cyclosporine A est d'au moins 3000 fois supérieure à celle de la Cyclosporine A.

En conséquence, les pro-drogues de la présente invention peuvent être facilement incorporées à des formulations galéniques aqueuses.

Egalement de façon remarquable, les pro-drogues de la présente invention s'avèrent ne pas être sensibles aux conditions de pH habituellement rencontrées pour ce genre d'application lorsqu'elles se trouvent en solution aqueuse.

De plus, les pro-drogues de la présente invention jouent pleinement leur rôle en libérant, avec un temps de demi-vie tout à fait approprié pour une application thérapeutique, la molécule pharmacologiquement active lorsqu'elles se trouvent au contact des enzymes présents dans les humeurs biologiques.

La présente invention concerne également une pro-drogue telle que décrite précédemment en tant que médicament.

Un tel médicament est de préférence utilisé pour le traitement des pathologies ou des conditions physiologiques nécessitant précédemment l'emploi d'une cyclosporine, en particulier de toutes les pathologies nécessitant l'emploi de la Cyclosporine A par voie locale ou par voie systémique intraveineuse.

Un tel médicament est en particulier destiné à permettre la prolongation de survie des allogreffes d'organes tels que le rein, le coeur, le foie, le pancréas, le poumon, l'intestin grêle, ou de la moelle osseuse. Il peut être destiné également à inhiber la réplication du virus de l'immunodéficience humaine de type 1 (VIH-1).

Dans de telles applications, la posologie de la pro-drogue de la présente invention en administration systémique intra-veineuse est telle que la concentration en cyclosporine générée lors du clivage, par exemple en Cyclosporine A, correspondant aux concentrations thérapeutiques habituellement préconisée.

De préférence encore, un tel médicament est utilisé dans le domaine ophtalmique et est destiné, en particulier, pour le traitement de pathologies de l'oeil et de ses annexes.

Parmi ces pathologies, on trouve, entre autres, les kératoconjonctivites sèches, appelées également syndrome de l'oeil sec, le syndrome de Sjögren, les kératoconjonctivites allergiques, en particulier, celles résistantes aux corticostéroïdes, les conjonctivites muco-synéchiantes, les kératites stromales herpétiques, les kératites limbiques immunitaires, la kératite de Thygeson, les pathologies liées aux greffes de cornée, et comme traitement adjuvant d'une chirurgie filtrante. De préférence encore, le médicament de la présente invention est utilisé pour le traitement des kératoconjonctivites sèches.

Dans de telles applications, la posologie de la pro-drogue de la présente invention est telle que la concentration lacrymale en cyclosporine générée lors du clivage de la pro-drogue, par exemple en Cyclosporine A, doit être supérieure à 0,5 µg/l en administration par voie locale.

Le médicament de la présente invention peut être administré, par voie topique, en particulier pour le traitement local d'affections des muqueuses ou d'affections cutanées, ou par voie parentérale, en particulier par voie intraveineuse. Il peut également être administré par voie orale dans le but d'améliorer la bio-disponibilité de la cyclosporine, par exemple de la Cyclosporine A.

Lorsque le médicament de la présente invention est administré par voie parentérale, les préparations galéniques appropriées peuvent être des solutions aqueuses concentrées et stériles, ou des poudres pour préparations injectables.

De préférence, le médicament de la présente invention est administré par voie intra-veineuse. Les préparations galéniques appropriées pour une telle administration sont des solutions aqueuses pour injection ou pour perfusion bien connues du spécialiste.

De préférence encore, le médicament de la présente invention est administré par voie locale. Les préparations galéniques appropriées pour une telle administration, en particulier pour une application ophtalmique, sont les collyres, sous la forme de solutions stériles aqueuses, les pommades ophtalmiques, les gels ophtalmiques, les inserts ophtalmiques

La présente invention, ainsi que ses propriétés avantageuses, sont présentées de façon détaillée, sans pour autant être limitative, dans les Exemples et avec l'aide du dessin dans lequel,
- la Figure 1 représente une courbe de cinétique de conversion in-vitro d'une pro-drogue selon l'invention par hydrolyse à l'aide d'estérases et une courbe de cinétique d'apparition de la Cyclosporine A;
- la Figure 2a représente le taux sanguin de Cyclosporine A après administration i.v. de la Cyclosporine A sous une forme huileuse chez le rat;
- les Figures 2b et 2c représentent le taux sanguin de Cyclosporine A après administration i.v. respectivement d'une solution aqueuse de deux des pro-drogues selon l'invention chez le rat; et
- la Figure 3 représente respectivement les concentration au cours du temps de la Cyclosporine A et d'une pro-drogue selon l'invention dans les larmes de lapin.

Dans la nomenclature utilisée dans les Exemples pour décrire les produits obtenus, le résidu de Cyclosporine A sera désigné par l'abréviation - CsA, le résidu du fragment opposé étant lié au seul groupe fonctionnalisable de ce undéca-peptide cyclique, à savoir le groupe hydroxyle de l'acide aminé ayant la position 1, la N-méthyl-(4R)-4-((E)-2-butényl)-4-méthyl-L-thréonine (MeBmt). Les formules chimiques développées des produits intermédiaires dérivés de la Cyclosporine A ne représenteront que le résidu d'acide aminé en position 1 avec la chaîne latérale respective.

### Exemple 1

### Préparation du undéca-peptide cyclique de formule (XV)

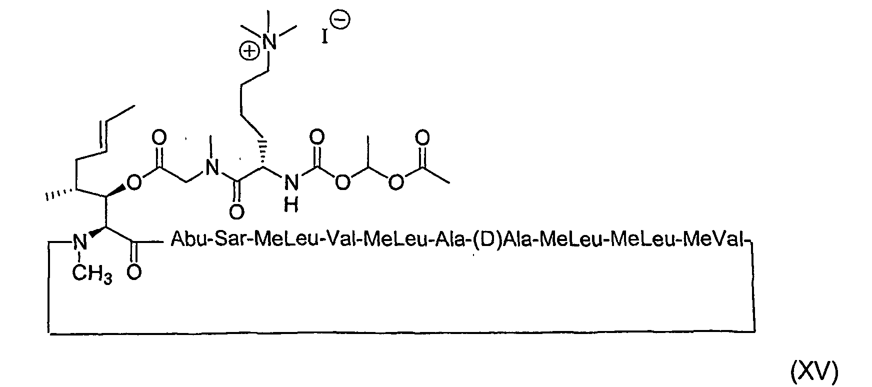

### 1 . Préparation de MeBmt(O-Sar-Lys(N_{ε}⁺Me₃)-COOCH(CH₃)-C-OCH₃))¹-CsA (XV)

### 1.1. Préparation du carbonate d'α-acétoxyéthyle d'oxycarbonyl-paranitrophénolate (2)

### 1.1.1. Préparation du carbonate d'α-chloroéthyle d'oxycarbonyl-paranitrophénolate (1)

A une solution de 3 g (21.6 mmol, 1 eq) de p-nitrophénol et 1.7 ml (21.7 mmol, 1 eq) de pyridine dans 108 ml de chloroforme sont ajoutés à 0°C, 2.6 ml (23.7 mmol, 1.1 eq) de chloroformiate d'α -chloroéthyle. Le mélange réactionnel est agité 30 min à 0°C puis 16h à température ambiante. Le mélange réactionnel est extrait à l'eau, avec une solution à 0.5% de NaOH puis à l'eau. La phase organique est séchée sur Na₂SO₄, filtrée et évaporée sous vide réduit pour donner une huile jaune qui donne un solide blanc pur (5.8 g) après cristallisation dans l'hexane.
RMN-¹H (400 MHz, CDCl₃): δ: 8.32 (d, 2H), 7.44 (d, 2H), 6.52 (q, 1H), 1.95 (s, 3H).

### 1.1.2. Préparation du carbonate d'α-acétoxyéthyle d'oxycarbonyl-paranitrophénolate (2)

A une solution de 4 g (16.3 mmol, 1 eq) de (1) dissous dans 100 ml d'acide acétique sont ajoutés 7.8 g (24.4 mmol, 1.5 eq) d'acétate de mercure. Le mélange réactionnel est agité pendant 1 jour à température ambiante puis 1 g (3.13 mmol, 0.2 eq) supplémentaire d'acétate de mercure est ajouté. Après 1 jour supplémentaire d'agitation à température ambiante, la réaction est complète. L'acide acétique est évaporé sous haut vide et le résidu repris dans l'éther. La phase organique est extraite avec une solution saline puis séchée sur Na₂SO₄, filtrée et évaporée sous vide réduit pour donner une huile jaune. Le produit brut est chromatographié sur gel de silice pour donner une huile incolore (4.4 g).
RMN-¹H (400 MHz, CDCl₃): δ: 8.31 (d, 2H), 7.43 (d, 2H), 6.87 (q, 1H), 2.16 (s, 3H), 1.64 (s, 3H).

### 1.2. Préparation de l' α-acétoxyéthoxycarbonyl-lysine(N_{ε}(Fmoc)) (5)

### 1.2.1 Préparation de l'ester benzylique de l'α-acétoxyéthoxycarbonyllysine(N_{ε}(Z)) (3)

500 mg (1.23 mmol, 1 eq) de H-Lys(Z)OBn.HCl sont mis en suspension dans 2.5 ml de dioxane. A température ambiante sont ajoutés 231 µl (1.35 mmol, 1.1 eq.) de N,N-diisopropyléthylamine (DIPEA) et 396 mg (1.47 mmol, 1.2 eq) de (2). Après 1 jour d'agitation à température ambiante, la réaction est complète. Le dioxane est évaporé sous vide réduit et le résidu repris dans 20 ml d'acétate d'éthyle. La phase organique est extraite trois fois avec une solution d'acide citrique à 6% (20 ml), une solution saturée de NaHCO₃ (20 ml) et une solution saturée en NaCl (20 ml), séchée sur Na₂SO₄ anhydre, filtré et le solvant est évaporé sous vide réduit. Le, produit brut obtenu est chromatographié sur gel de silice pour donner une huile claire, transparent (576 mg).
ESI-MS: m/z: 501.34 [M+H⁺]; 518.28 [M+H₂O+H⁺].

### 1.2.2. Préparation de l'α-acétoxyéthoxycarbonyl-lysine (4)

A une solution de 509 mg (1.02 mmol) de (3) dans 10 ml d'éthanol sont ajoutés 50 mg de palladium sur charbon actif. Après 3 h d'agitation à température ambiante sous flux d'hydrogène, la réaction est complète. Le mélange réactionnel est filtré sur célite et le filtrat évaporé sous vide réduit pour donner le brut sous forme de cristaux brunâtres qui est engagé directement dans l'étape suivante (254 mg).
ES-MS m/z: 276.87 [M+H⁺].

### 1.2.3 Préparation de l'α-acétoxyéthoxycarbonyl-lysine(N_{ε}(Fmoc)) (5)

A une solution de 1.062 (3.84 mmol, 1 eq) de (4) dissous dans 38 ml de dioxane sont ajoutés 1.11 ml de DIPEA (6.53 mmol, 1.7 eq) et 1.555 g (4.61 mmol, 1.2 eq) de Fmoc-O-Suc. Après 1 h d'agitation à température ambiante, la réaction est complète, le dioxane est évaporé sous vide réduit et le résidu repris dans 20 ml d'AcOEt. La phase organique est lavée une fois avec une solution d'acide citrique 6% (20 ml) et une solution saturée en NaCl (20 ml), séchée sur Na₂SO₄ anhydre, filtrée et le solvant est évaporé sous vide réduit. Le produit brut obtenu est chromatographié sur gel de silice pour donner une mousse blanche (1.026 g).
ES-MS m/z: 499.37 [M+H⁺], 516.29 [M+H₂O+H⁺].

### 1.3. Préparation de MeBmt(O-Sar-Lys(N_{ε}⁺Me₃)-COOCH(CH₃)OCOCH₃))¹-CsA.I⁻ (XV)

### 1.3.1 Préparation de MeBmt(O-COCH₂Br)¹-CsA (6)

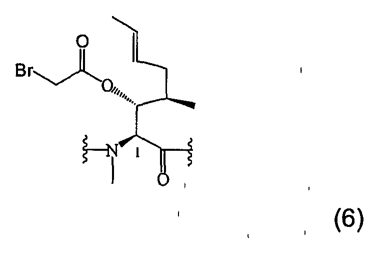

4 g (3.33 mmol, 1 eq) de CsA sèche sont dissous sous argon dans 66 ml (0.76 mol) de bromure de bromoacétyle. Par petites portions, sont ajoutés 2g (16.64 mmol, 5 eq) de diméthylaminopyridine et le mélange réactionnel est agité à température ambiante pendant 40 min. La réaction est alors complète. Le mélange réactionnel est versé, avec précaution et sous agitation vigoureuse, dans un mélange d'hydrogénocarbonate (77 g, 0.91 mol), d'eau (500 ml) et de glace pillée. L'addition éventuelle de quelques portions supplémentaires de NaHCO₃ permet d'amener le pH de la solution à 7-8. La phase aqueuse séparée est extraite deux fois au dichlorométhane et les phases organiques réunies sont extraites trois fois avec une solution saturée de NaHCO₃ et avec une solution saturée de NaCl, séchées sur Na₂SO₄ anhydre, filtrées et le solvant est évaporé sous vide réduit. Le produit brut obtenu est chromatographié sur gel de silice pour donner une mousse blanche (3.2 g).
ESI-MS : m/z : 622.6 [M+2H⁺], 673.9 [M+Na⁺+H⁺].

### 1.3.2. Préparation de MeBmt(O-Sar-H)¹-CsA (7)

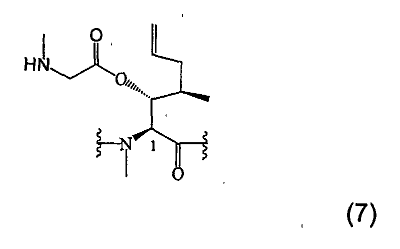

300 mg (0.23 mmol, 1 eq) de (6) sont dissous dans 2.3 ml d'éthanol. Sont ajoutés à température ambiante, 95 µl (0.68 mmol, 3 eq) de triéthylamine (TEA) et 31 mg (0.45 mmol, 2 eq) du chlorure de méthylammonium supplémentaire. Après 3 jours d'agitation à température ambiante, ajustement du pH à 12 par ajout de TEA et addition de 15 mg (0.23 mmol, 1 eq) de chlorure de méthylammonium, la réaction est complète après 1 heure. L'éthanol est évaporé sous vide réduit et le résidu est repris dans de l'acétate d'éthyle. La phase organique est extraite à l'eau et avec une solution saturée en NaCl., séchées sur Na₂SO₄ anhydre, filtrées et le solvant est évaporé sous vide réduit. Le produit brut obtenu est chromatographié sur gel de silice pour donner une mousse blanche (200 mg).
ESI-MS : m/z : 1273.7 [M+H⁺].

### 1.3.3 Préparation de MeBmt(O-Sar-Lys(N_{ε}(FMOC)-COOCH(CH₃)OCOCH₃))¹ -CsA (8)

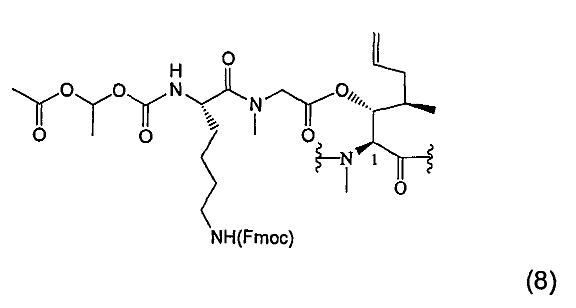

79 mg (0.06 mmol, 1 eq) de (7) sont dissous dans 0.5 ml de dichlorométhane (DCM) sous argon. Sont ajoutés successivement sous argon 31.6 µl (0.18 mmol, 3 eq) de DIPEA, 35 mg (0.09 mmol, 1.5 eq) de hexafluorophosphate de O-(7-azabenzotriaxol-1-y1)-1,1,3,3-tétraméthyluronium (HATU) et 40 mg (0.08 mmol, 1.3 eq) de (5) dissous dans 0.8 ml DCM. Après 3 h d'agitation à température ambiante, la réaction est complète. Le DCM est évaporé sous vide réduit et le résidu repris dans 20 ml d'AcOEt. La phase organique est lavée une fois avec une solution d'acide citrique à 6% (20 ml), une solution saturée de NaHCO₃ (20 ml) et une solution saturée de NaCl (20 ml), séchée sur Na₂SO₄ anhydre, filtrée et le solvant évaporé sous vide réduit. Le produit brut est chromatographié sur gel de silice pour donner une mousse blanche (59 mg).
ES-MS m/z: 1754.36 [M+H⁺]; 877.86 [M+2H⁺].

### 1.3.4 Préparation de MeBmt(O-Sar-Lys(N_{α}-COOCH(CH₃)OCOCH₃))¹-CsA (9)

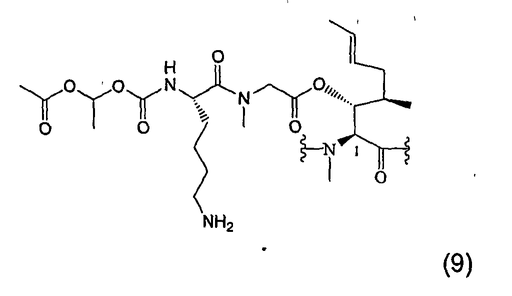

A une solution de 150 mg (0.09 mmol, 1 eq) de (8) dissous dans 900 µl d'acétonitrile sont ajoutés 90 µl (0.86 mmol, 10 eq) de diéthylamine. Après 3 h d'agitation à température ambiante, la réaction est complète. Le solvant est évaporé sous vide réduit et le produit brut est chromatographié sur gel de silice pour donner une mousse blanche (52 mg).
ES-MS m/z: 1532.79 [M+H⁺]; 766.79 [M+2H⁺].

### 1.3.4. Préparation de MeBmt(O-Sar-Lys(N_{ε}⁺Me₃)-COOCH(CH₃)OCOCH₃))¹ -CsA.I⁻ (XV)

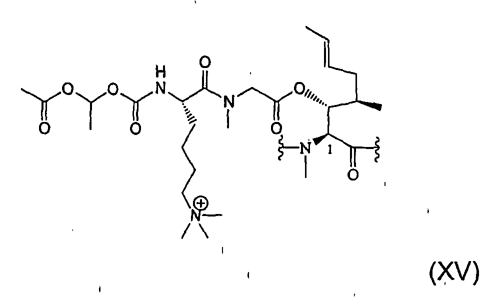

49 mg (0.03 mmol, 1 eq) de (9) sont dissous dans 640 µl de DCM anhydre, puis 30 µl (0.48 mmol, 15 eq) de Mel suivis de 14 µl (0.08 mmol, 2.5 eq) de DIPEA sont additionnés. Après 1 jour d'agitation à température ambiante la réaction est complète. Le DCM est évaporé sous vide réduit et le produit brut est purifié par HPLC semi-préparative pour isoler le composé pur sous forme de lyophilisat (30 mg).
ES-MS m/z: 1574.37 [M+H⁺], 787.83 [M+2H⁺].

### Exemple 2

### Préparation du undéca-peptide cyclique de formule (XVI)

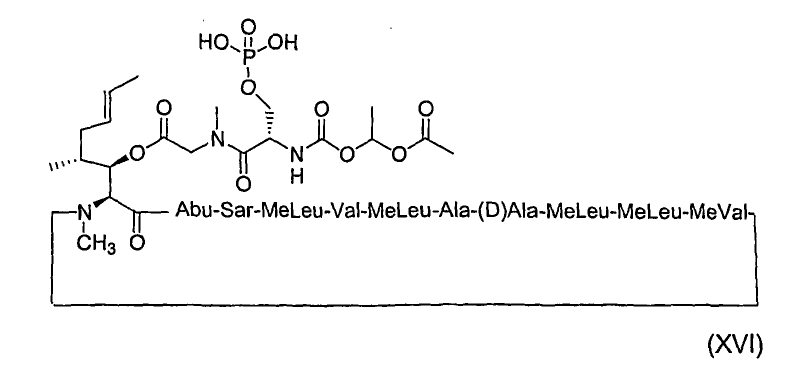

### 1. Préparation de MeBmt(O-Sar-Ser(OPO(OH)₂)-COOCH(CH₃)OCOCH₃)¹ -CsA. (XVI)

### 1.1. Préparation de l' α-acétoxyéthoxycarbonyl-sérine (11)

### 1.1.1. Préparation de l'ester benzylique de l'α-acétoxyéthoxycarbonyl-sérine (10)

1.4 g (6.04 mmol, 1 eq) de H-Ser-OBn.HCl sont mis en suspension dans 12 ml de dioxane. A température ambiante, sont ajoutés 1.14 ml (6.64 mmol, 1.1 eq) de DIEA et 2.1 g (7.85 mmol, 1.3 eq) de (2) obtenu selon l'Exemple 1. Après une nuit d'agitation à température ambiante, la réaction est complète. Le dioxane est évaporé sous vide réduit et le résidu repris dans de l'acétate d'éthyle. La phase organique est extraite trois fois avec une solution d'acide citrique à 6%, une solution saturée de NaHCO₃ et une solution saturée en NaCl, séchée sur Na₂SO₄ anhydre, filtrée et le solvant est évaporé sous vide réduit. Le produit brut obtenu est chromatographié sur gel de silice pour donner une mousse blanche (1.7 g).
RMN-¹H (400 MHz, CDCl₃): δ: 7.34-7.41 (m, 5H), 6.79-6.85 (m, 1H), 5.72-5.82 (m, 1 H), 5.24 (s, 2H), 4.47 (m, 1 H), 3.94-4.05 (m, 2H), 2.06 et 2.08 (s, 3H), 1.49 et 1.50 (d, 3H).

### 1.1.2. Préparation de l' α-acétoxyéthoxycarbonyl-sérine (11)

A une solution de 400 mg (1.23 mmol) de (10) dans 13 ml d'éthanol sont ajoutés 40 mg de palladium sur charbon actif. Après 4h d'agitation à température ambiante sous flux d'hydrogène, la réaction est complète. Le mélange réactionnel est filtré sur célite et le filtrat évaporé sous vide réduit pour donner le brut sous forme de dépôt translucide qui est engagé directement dans l'étape suivante (328 mg).
RMN-¹H (400 MHz, CDCl₃): δ:. 6.3-6.8 (m, 1 H), 5.8-6.3 (m, 1H), 4.3-4.4 (m, 1 H), 3.7-4.1 (m, 2H), 2.08 (s), 1.49 et 1.50 (d, 3H).

### 1.2. Préparation de MeBmt(O-Sar-Ser(OPO(OH)₂)-COOCH(CH₃)OCOCH₃)¹ -CsA, (XVI)

### 1.2.1 Préparation de MeBmt(O-Sar-Ser-COOCH(CH₃)OCOCH₃)¹-CsA (12)

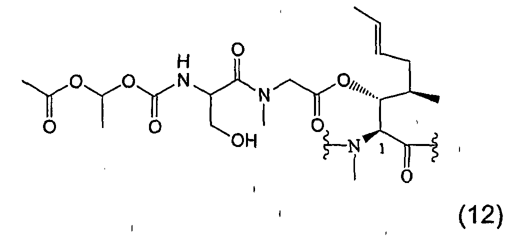

170 mg (0.13 mmol) de (7) obtenu selon l'Exemple 1 sont dissous dans 3 ml de dichlorométhane sous argon. Sont ajoutés successivement sous argon, 92 µl (0.53 mmol, 4 eq) de DIEA, 51 mg (0.26 mmol, 2 eq) de HATU et 60 mg de (11) (0.26 mmol, 2 eq). Après 5 heures d'agitation à température ambiante, la réaction est complète. Le dichlorométhane est évaporé sous vide réduit et le résidu repris dans de l'acétate d'éthyle. La phase organique est extraite trois fois avec une solution d'acide citrique à 6%, une solution saturée de NaHCO₃ et une solution saturée en NaCl, séchée sur Na₂SO₄ anhydre, filtrée et le solvant est évaporé sous vide réduit. Le produit brut obtenu est chromatographié sur gel de silice pour donner une mousse blanche (143 mg).
ESI-MS : m/z : 1513.32 [M+Na⁺], 1508.34 [M+H₂O+H⁺], 1491.36 [M+H⁺], 746.36 [M+2H⁺].

### 1.2.2. Préparation de MeBmt(O-Sar-Ser(OPO(OAll)₂)-COOCH(CH₃)OCOCH₃)¹-CsA (13)

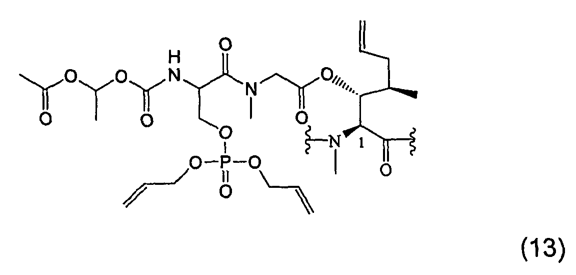

130 mg (0.09 mmol, 1 eq) de (12) sont dissous dans 880 µl de CH₂Cl₂ anhydre. Puis 20 mg (0.27 mmol, 3 eq) de 1H-tetrazole suivis de 52 µl (0.17 mmol, 2 eq) de (AllO)₂PN(iPr)₂ sont additionnés. Après 4 h d'agitation à température ambiante, le mélange réactionnel est refroidi à -60°C, 44 mg (0.17 mmol, 2 eq) d'acide m-chloroperbenzoïque sont ajoutés et l'agitation est poursuivie 30 min à -60°C, 15 min à 0°C et 45 min à température ambiante. A 0°C, 0.5 ml d'une solution à 10% de Na₂S₂O₅ sont ajoutés au milieu réactionnel, afin de détruire l'excès d'oxydant puis on extrait au dichlorométhane. La phase organique est lavée avec une solution à 10% de Na₂S₂O₅ puis le dichlorométhane est évaporé sous vide réduit. Le résidu est repris dans l'éther de méthyle et tertiobutyle et cette phase organique extraite avec une solution d'acide citrique à 6%, une solution saturée en NaCl, séchée sur Na₂SO₄ anhydre, filtrée et le solvant est évaporé sous vide réduit. Le produit brut obtenu est chromatographié sur gel de silice pour donner une mousse blanche (98 mg).
ESI-MS : m/z: 1651.38 [M+H⁺], 826.35 [M+2H⁺].

### 1.2.3. Préparation de MeBmt(O-Sar-Ser(OPO(OH)₂)-COOCH(CH₃)OCOCH₃)¹. -CsA (XVI)

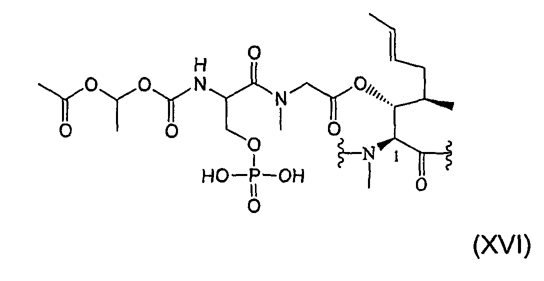

A une solution de 184 mg (0.58 mmol, 3 eq) de Bu₄N+·F⁻·H₂O0 dans 2 ml de CH₂Cl₂ sont ajoutés sous argon et à température ambiante 206 µl (1.55 mmol, 8 eq) de Me₃SiN₃ et 1.12 g (0.97 mmol, 5 eq) de (PPh₃)₄Pd°. Après agitation à température ambiante pendant dix minutes, 320 mg (0.19 mmol, 1 eq) de (13) sont ajoutés et le mélange réactionnel laissé agiter à température ambiante pendant trente minutes. La réaction est alors complète. Le mélange réactionnel est hydrolysé par addition d'une solution d'acide citrique à 6% et le dichlorométhane est évaporé sous vide réduit. Le résidu est repris dans de l'acétate d'éthyle et la phase organique obtenue est extraite trois fois avec une solution d'acide citrique à 6% et une solution saturée en NaCl, séchée sur Na₂SO₄ anhydre, filtrée et le solvant est évaporé sous vide réduit. Le produit brut obtenu est chromatographié sur cartouche Sep-Pack® puis sur HPLC préparative pour isoler le composé pur sous forme de lyophilisat (342 mg).
ESI-MS : m/z : 1593.32 [M+Na⁺], 1571.81 [M+H⁺].

### Exemple 3

### Propriétés physico-chimiques des undéca-peptides cycliques de formules (XV) et (XVI)

### 1. Hydrosolubilité des undéca-peptides cycliques de formules (XV) et (XVI)

Les solubilités dans l'eau ont été évaluées par un examen visuel à température ambiante du laboratoire par dissolution directe d'une quantité pesée du undéca-peptide cyclique dans des tampons phosphates 67 mM de type Sorensen. Les valeurs sont rassemblées dans le Tableau 1.

**Tableau 1**

| Undéca-peptide cyclique | Solubilité aqueuse à | | | |
|---|---|---|---|---|
| | pH=5 | pH=6 | pH=7 | pH=8 |
| (XV) | | | >7 mM | |
| (XVI) | >20 mM | >20 mM | >20 mM | >20 mM |

A titre indicatif, la Cyclosporine A est décrite pour avoir une solubilité maximale dans l'eau de 33 µg/ml à une température de 20°C et à pH 7, ce qui correspond à une concentration maximale de 0,027 mM.

### 2. Stabilité chimique et enzvmatique des undéca-peptides cycliques de formules (XV) et (XVI)

Une première évaluation de la stabilité chimique au cours du temps du undéca-peptide cyclique de formule (XV) a été effectuée, d'une part dans une solution isotonique constituée de mannitol, d'autre part dans un tampon phosphate (PBS) à un pH de 7 et à des températures de 4, 20 et 37°C.

Les pourcentages de Cylosporine A détectée sont rassemblés dans le Tableau 2 suivant:

**Tableau 2**

| | 4°C, mannitol | 20°C, mannitol | 37°C, mannitol | 37°C, PBS |
|---|---|---|---|---|
| 30 jours | 3,5% | 17,0% | 25,0% | 90,0% |
| 90 jours | 5,0% | 38,0% | | |

Comme ont peut le constater, à la température de 4°C dans une solution de mannitol, le peptide cyclique s'avère être stable répondre pendant au moins 90 jours.

Une deuxième étude de stabilités, à la fois chimique et enzymatique, a été effectuée avec les deux undéca-peptides cycliques de formule (XV) et (XVI) mis en solution dans un tampon HEPES 50 mM de pH 7,4 à 37°C, en absence et en présence d'estérases. Durant l'incubation à 37°C, des aliquotes de 40 µl sont prélevés à des intervalles de temps appropriés et analysés par HPLC et ESI-MS.

En absence d'enzyme, une stabilité chimique supérieure à 3 jours est observée pour les deux undéca-peptides cycliques.

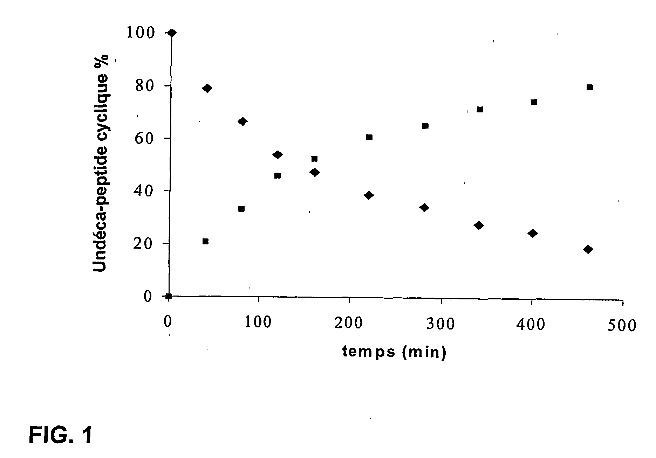

Les résultats obtenus lors de l'hydrolyse en présence d'estérases ont été rassemblés dans la Figure 1. La courbe de cinétique de conversion du undéca-peptide cyclique (XVI) est représentée par des losanges alors que la courbe de cinétique d'apparition de la Cyclosporine A est représentée par de carrés. Comme on peut le constater selon cette figure, en présence d'estérases, le undéca-peptide cyclique (XVI) se dégrade rapidement pour libérer la Cyclosporine A. Une observation similaire a été faite pour le undéca-peptide cyclique (XV).

Les undéca-peptides cycliques (XV) et (XVI) ont été soumis à une incubation dans du sérum bovin à 37°C. Les temps de demi-vie de conversion des undéca-peptides cycliques en Cyclosporine A ont été évalués et sont respectivement de 3,66 et 3,50 heures.

### Exemple 4

### Application pour administration intra-veineuse sous forme de solution aqueuse Etude de pharmacocinétique des undéca-peptides cycliques de formules (XV) et (XVI)

Afin de mettre à disposition du clinicien un outil alternatif aux formulations galéniques de la Cyclosporine A couramment utilisé sous la forme de micro-émulsion dans de l'huile de ricin polyoxyéthylénée de stabilité délicate, peu facile à manipuler et induisant des effets délétères, des pro-drogues de la présente invention, sous la forme de simples solutions aqueuses, ont été évaluées en vue d'une application intra-veineuse.

Ainsi, les deux undéca-peptides cycliques (XV) et (XVI), en solution dans du tampon phosphate, ont été administrés par voie intraveineuse chez le rat à raison d'une dose équivalente à 10 mg/kg de Cyclosporine A.

En référence, un échantillon d'une solution injectable de Cyclosporine, disponible dans le commerce sous la marque de commerce Sandimmun a été utilisé après dilution appropriée.

Des prélèvements de sang ont été effectués à intervalles réguliers puis soumis à analyse en vue d'effectuer un dosage de la Cyclosporine A.

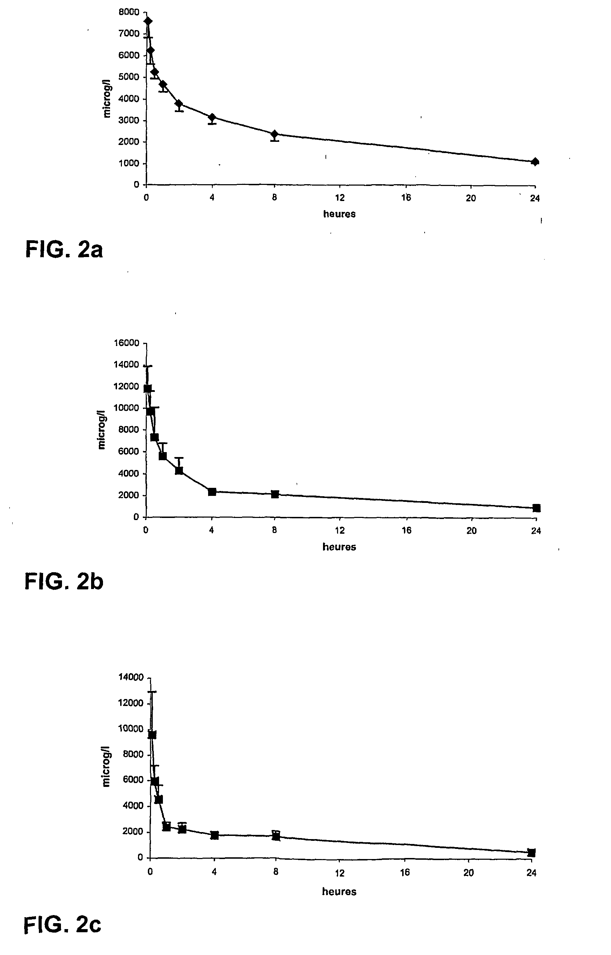

Les taux sanguin de Cyclosporine A après administration i.v. de la Cyclosporine A ont été rassemblés sur la Figure 2a. Ceux obtenus après administration i.v. respectivement d'une solution aqueuse des deux pro-drogues (XV) et (XVI) sont rassemblés respectivement sur les Figures 2b et 2c. Les résultats interprétés des courbes des Figures 2a, 2b et 2c ont été rassemblés dans le Tableau 3 suivant.

**Tableau 3**

| Undéca-peptide | Cyclosporine A | (XV) | (XVI) |
|---|---|---|---|
| AUC_{0-inf}µg.h.L⁻¹ | 69229 | 66626 | 44699 |
| CLL/h/kg | 0,14 | 0,15 | 0,22 |
| MRT h | 95,2 | 15,3 | 14,9 |
| Vss L/kg | 2,2 | 2,2 | 3,4 |
| T1/2 ₁ h | 0,31 | 0,55 | 0,20 |
| T1/2 ₂ h | 11,1 | 11,8 | 11,8 |

Dans ce tableau, la signification des abréviations est la suivante:
- AUC: aire sous la courbe ("Area under the curve");
- CL: clairance ("Clearance");
- MRT: ("Mean residence time");
- Vss: volume de distribution à l'état d'équilibre ("Volume of distribution at steady state");
- T1/2 ₁: temps de demi-vie initiale; et
- T1/2 ₂: temps de demi-vie terminale.

Comme on peut le constater de la Figure 2 et du Tableau 3, les paramètres pharmacocinétiques de l'expérience contrôle avec la Cyclosporine A sont comparables à ceux rapportés dans la littérature. L'aire sous la courbe de la Cyclosporine A générée lors du clivage du undéca-peptide cyclique (XV) est comparable à celle de la Cyclosporine A de l'expérience contrôle alors que celle de la Cyclosporine A générée lors du clivage du undéca-peptide cyclique (XVI) est réduite de 25% par rapport à celle de la Cyclosporine A de l'expérience contrôle.

Les deux undéca-peptides cycliques (XV) et (XVI) montre chacun un profil de libération sanguine de la Cyclosporine A similaire. Ces profils sont similaires à celui de la Cyclosporine A sous la forme galénique à base d'huile de ricin polyoxyéthylénée.

De ces résultats, il en résulte que les pro-drogues de la présente invention offre, pour un profil de libération de Cyclosporine A équivalent, les grands avantages suivants par rapport aux formulations galéniques existantes de Cyclosporine A:
- facilité de mise en oeuvre par simple dissolution dans l'eau; et
- pas d'obligation d'utiliser des excipients s'avérant être toxiques; et
- pas d'obligation pour utiliser des matériaux particulier pour les manipuler.

### Exemple 5

### Application pour administration topique oculaire sous forme de solution aqueuse

### Etude de pharmacocinétique du undéca-peptide cyclique de formule (XV)

Afin de mettre à disposition du clinicien un outil permettant une administration topique oculaire de Cyclosporine A sans que des irritations ou des sensation désagréable soient ressenties ou que la vision ne se trouble, des pro-drogues de la présente invention, sous la forme de simples solutions aqueuses, ont été évaluées.

### 1. Préparation des solutions

Des solutions aqueuses isotoniques à 5% de mannitol et à pH 7,0 du undéca-peptide cyclique (XV) ont été préparées. La concentration en équivalent en Cyclosporine A est de 1 % (poids/volume). Les solutions ont été stérilisées par passage au travers de filtres de nitrocellulose à 0,22 µm. Une formulation de référence de Cyclosporine A a été préparée sous la forme d'une solution à 1 % dans de l'huile d'olive.

### 2. Détermination de la tolérance du undéca-peptide cyclique de formule (XV)

La tolérance oculaire a été déterminée selon deux méthodes, à savoir selon le test modifié de Draize et par un ophtalmoscope à balayage laser confocal.

### 2.1. Test modifié de Draize (test de tolérance aiguë)

Cette évaluation a été opérée sur six lapins mâles albinos. Pour chaque animal, un oeil a reçu une instillation de 50 µl de la solution telle que décrite précédemment, l'autre oeil, non traité, jouant le rôle de contrôle.

L'évaluation clinique d'une éventuelle irritation a été effectuée visuellement par évaluation de l'écoulement oculaire, du chémosis conjonctival et de la rougeur conjonctivale selon la classification décrite dans le Tableau 4 suivant:

**Tableau 4**

| | | |
|---|---|---|
| Ecoulement | Normal | 0 |
| oculaire | Ecoulement faible | 1 |
| | Ecoulement sévère couvrant une petite surface de la cornée | 2 |
| | Ecoulement sévère couvrant une grande partie de la cornée | 3 |
| Chémosis | Normal | 0 |
| | Chémosis faible incluant la membrane nictitante | 1 |
| | Chémosis sévère avec l'oeil partiellement clos | 2 |
| | Chémosis sévère avec l'oeil clos | 3 |
| Rougeur | Vaisseaux sanguins normaux | 0 |
| | Certains vaisseaux hyperhémiques | 1 |
| | Rougeur diffuse, vaisseaux individuels non facilement discernables | 2 |
| | Rougeur forte diffuse | 3 |

L'éventuelle irritation a été observée, sur chaque animal, selon les règles précédentes, à intervalles déterminés s'échelonnant sur 48 heures après l'instillation et un indice d'irritation total (Iᵢᵣᵣ) a été calculé en procédant à la somme totale des indices estimés. Les résultats obtenus sont rassemblés dans le Tableau 4 au point 2.3 ci-dessous.

### 2.2. Ophtalmoscopie à balayage laser confocal ("Confocal laser scanning opthalmoscope, CLSO") (test d'évaluation d'une toxicité sub-aiguë sur 4 jours d'administration)

Ce test a été effectué sur le même type d'animal que précédemment. 25 µl d'une solution telle que décrite précédemment,ont été instillés sur la cornée de l'oeil droit, trois fois par jour pendant quatre jours puis une fois au quatrième jour juste avant l'observation. Après la dernière instillation, les lapins ont été mis sous sédation par administration de chlorhydrate de kétamine et de xylazine. Un total de 25 µl d'une solution de fluorescéine de sodium à 0,5% en poids/volume ont été instillés sur l'oeil afin de permettre un marquage sélectif des surfaces éventuellement endommagées. L'oeil a ensuite été rincé pendant une minute par une solution saline à 37°C.

Enfin, l'oeil a été observé par un ophtalmoscope à balayage laser confocal selon la méthode décrite par Furrer et al., J. Ocular Pharmacol., 1997, 13, 559. L'ophtalmoscope a été couplé à un système de traitement d'image afin de pouvoir reconstituer une image en trois dimensions et permettre une évaluation des zones endommagées.

Le degré de tolérance est évalué en fonction du pourcentage de lésions de cornée et selon la règle suivante:
- de 0% à 25%: bonne tolérance;
- de 25% à 40%: tolérance acceptable;
- de 40% à 60%: faible tolérance; et
- au dessus de 60%: tolérance inacceptable.

On note qu'il est généralement reconnu qu'un pourcentage de lésions inférieur ou égal à 5% correspond à un taux de mortalité cellulaire habituel chez un corps sain non soumis à un quelconque traitement.

Les résultats obtenus sont rassemblés dans le Tableau 5 au point 2.3. ci-dessous.

### 2.3. Résultats de la tolérance oculaire du undéca-peptide cyclique de formule (XV)

**Tableau 5**

| | Undéca-peptide (XV) | Cyclosporine A |
|---|---|---|
| Draize, Iᵢᵣᵣ | 1,8 | 1,9 |
| CLSO, % de lésions | 7 | 23 |

Du Tableau 5, il apparaît qu'un indice d'irritation total de 1,8 a été obtenu au test de Draize pour le undéca-peptide cyclique de formule (XV) et que 7% de la cornée ont été lésées par administration de ce produit. Ces deux résultats démontrent une très bonne tolérance à ce undéca-peptide cyclique, cette tolérance étant nettement améliorée par rapport à celle obtenue lorsque la Cyclosporine A est administrée dans de l'huile d'olive.

Pour des raisons évidentes, l'amélioration subjective du confort de la vision par l'utilisation d'une solution aqueuse plutôt que huileuse n'a pas été évaluée sur l'animal.

### 3. Stabilité du undéca-peptide cyclique de formule (XV)

Des échantillons des solutions décrites précédemment ont été conservés respectivement à 4°C et 20°C. Des analyses par HPLC ont été effectuées régulièrement pendant 3 mois. Ces échantillons se sont avérés posséder une bonne stabilité dans de telles conditions.

### 4. Cinétique de conversion ex-vivo du undéca-peptide cyclique de formule (XV)

Ce test de cinétique de conversion a été effectué en incubant à 37°C 25 µl d'un échantillon de la solution décrite précédemment sous légère agitation avec 8 µl de larme fraîche de lapin. A intervalles de 1, 2, 3 et 30 minutes, des prélèvements de 2 µl ont été effectués puis analysés par HPLC.

Les résultats obtenus ont montré que le undéca-peptide cyclique de formule (XV) joue son rôle de pro-drogue dès la première minute de contact avec la larme de lapin en libérant la Cyclosporine A. A 3 minutes, 3% de la pro-drogue se trouvent convertis, puis 4,7% au bout de 30 minutes.

### 5. Cinétique de conversion in-vivo du undéca-peptide cyclique de formule (XV)

Ce test de cinétique de conversion in-vivo a été effectué en instillant un échantillon de 25 µl de la solution précédemment décrite dans l'oeil droit de lapins mâles albinos (4 kg). Des échantillons de larmes ont été prélevés à intervalles de 1, 2, 3, 4 et 20 minutes puis analysés par HPLC.

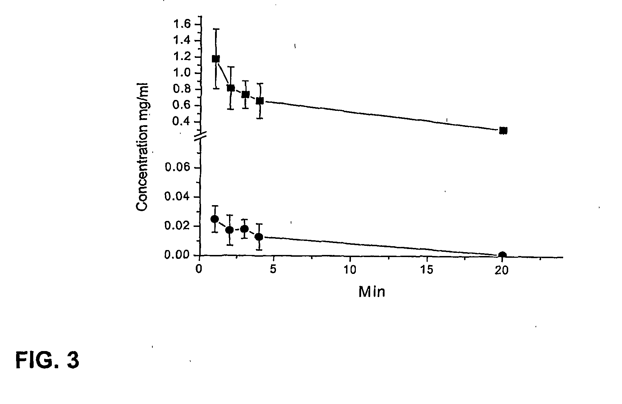

Les résultats obtenus ont été rassemblés sur la Figure 3. Comme on peut le constater, ce test vient confirmer les résultats déjà obtenus lors de l'expérience ex-vivo. Le undéca-peptide cyclique de formule (XV) (carrés) joue bien son rôle de pro-drogue dès la première minute de contact avec la larme de lapin en libérant la Cyclosporine A (ronds) et cette libération se poursuit au cours des 20 minutes qui suivent. A 1 minute, la concentration en Cyclosporine A dans la larme est de 0,025 mg/ml.

### 6. Conclusions

De ces résultats, il en résulte que les pro-drogues de la présente invention offre, pour une administration topique oculaire, les avantages suivants:
- facilité de préparation d'une formulation galénique telle qu'un collyre par simple dissolution dans une solution aqueuse sans nécessité d'avoir recours à des adjuvants huileux;
- bonne tolérance aiguë et très bonne tolérance sub-aiguë supérieures à celles obtenue avec une formulation galénique de cyclosporine sous forme huileuse;
- bonne stabilité; et
- temps de demi-vie approprié pour une application ophtalmique.

## Revendications

1. Pro-drogue constitué d'un undéca-peptide cyclique dont l'enchaînement peptidique comprend au moins un résidu d'acide aminé de formule générale (I) suivante: 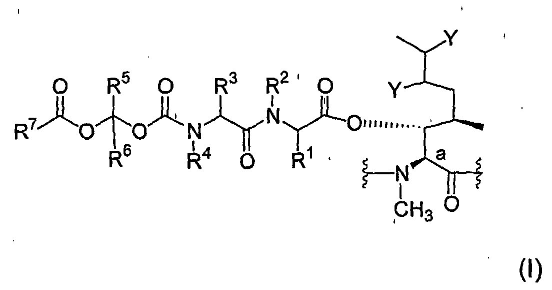 dans laquelle:
- l'atome de carbone C^{a} constitue l'un des maillons du cycle undéca-peptidique ;
- les substituants Y représentent chacun un atome d'hydrogène ou constituent ensemble une liaison;
- les substituants R¹ et R³ représentent, indépendamment l'un de l'autre, un atome d'hydrogène, un groupe aralkyle, un groupe alkaryle, un groupe hétéroalkyle, un groupe hétérocyclique, un groupe alkyl-hétérocyclique, un groupe hétérocyclique-alkyle ou un groupe alkyle ayant de 1 à 6 atomes de carbone, linéaire ou ramifié, et éventuellement substitués par au moins un des groupements choisis parmi -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH)₂, et les différentes formes salifiées de ces groupements, chacun des substituants R⁸ représentant indépendamment l'un de l'autre un groupe alkyle, linéaire ou ramifié, ayant de 1 à 6 atomes de carbone;
- les substituants R² et R⁴ représentent, indépendamment l'un de l'autre, un atome d'hydrogène, un groupe alkaryle, ou un groupe alkyle ayant de 1 à 6 atomes de carbone, linéaire ou ramifié;
- les substituants R⁵ et R⁶ représentent, indépendamment l'un de l'autre, un atome d'hydrogène, un groupe aralkyle, un groupe alkyle ayant de 1 à 6 atomes de carbone, linéaire ou ramifié; et
- le substituant R⁷ représente un groupe aralkyle, un groupe alkaryle, un groupe hétéroalkyle, un groupe hétérocyclique, , un groupe alkyl-hétérocyclique, un groupe hétérocyclique-alkyle ou un groupe alkyle ayant de 1 à 6 atomes de carbone, linéaire ou ramifié lesdits groupes étant éventuellement substitués par au moins un des groupements choisis parmi -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH)₂, et les différentes formes salifiées de ces groupements, chacun des substituants R⁸ ayant la définition précédente.

2. Pro-drogue selon la revendication 1, **caractérisée en ce que**, dans formule générale (I) définissant ledit résidu d'acide aminé, au moins l'un des substituants R¹ et R³ représente un groupe aralkyle, un groupe alkaryle, un groupe hétéroalkyle, un groupe hétérocyclique, un groupe alkyl-hétérocyclique, un groupe hétérocyclique-alkyle ou un groupe alkyle ayant de 1 à 6 atomes de carbone, linéaire ou ramifié, chacun desdits groupes étant substitué par au moins un des groupements choisis parmi -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH)₂, et les différentes formes salifiées de ces groupements, chacun des substituants R⁸ ayant la définition précédente.

3. Pro-drogue selon la revendication 2, **caractérisée en ce que** lesdits groupes aralkyle, alkaryle, hétéroalkyle, hétérocyclique, alkyl-hétérocyclique, hétérocyclique-alkyle ou alkyle sont substitués par au moins un des groupements choisis parmi -NR⁸₂, -N⁺R⁸₃, -OPO(OH)₂ ou ses différentes formes salifiées de ces groupements, chacun des substituants R⁸ ayant la définition précédente.

4. Pro-drogue selon la revendication 3, **caractérisée en ce que** au moins l'un desdits substituants R¹ et R³ représentent un groupe alkyle linéaire ayant de 1 à 6 atomes de carbone substitués par au moins un des groupements choisis parmi -NR⁸₂, -N⁺R⁸₃, -OPO(OH)₂ ou ses différentes formes salifiées de ces groupements, chacun des substituants R⁸ ayant la définition précédente.

5. Pro-drogue selon la revendication 1, **caractérisée en ce que**, dans formule générale (I) définissant ledit résidu d'acide aminé, les substituants R⁵ et R⁶ ne peuvent représenter simultanément un atome d'hydrogène.

6. Pro-drogue selon la revendication 1, **caractérisée en ce que**, dans formule générale (I) définissant ledit résidu d'acide aminé, au moins l'un desdits substituants R⁵ et R⁶ représente un groupe alkyle ayant de 1 à 6 atomes de carbone, linéaire ou ramifié, et le substituant R⁷ représente un groupe aralkyle ou un groupe alkyle ayant de 1 à 6 atomes de carbone, linéaire ou ramifié.

7. Pro-drogue selon la revendication 6, **caractérisée en ce que** lesdits substituants R⁵ et R⁶ représentent, indépendamment l'un de l'autre, un atome d'hydrogène ou un groupe méthyle.

8. Pro-drogue selon la revendication 1, **caractérisée en ce que** ledit enchaînement peptidique comprend un seul résidu d'acide aminé de formule générale (I) formant un cycle undéca-peptidique avec une séquence linéaire de dix acides aminés de formule générale (II) suivante:
-T-U-V-W-MeLeu-Ala-X-MeLeu-Z-MeVal- (II)
dans laquelle:
- T est choisi parmi les acides aminés Ala, Abu, Nval, Val et Thr;
- U est choisi parmi les acides aminés Sar, (D)MeSer, (D)MeAla et (D)MeSer(OCOR⁹), avec R⁹ représentant un atome d'hydrogène, un groupe alkaryle, ou un groupe alkyle ayant de 1 à 6 atomes de carbone, linéaire ou ramifié;
- V représente un acide aminé de formule générale (N-R¹⁰)aa, aa étant choisi parmi les acides aminés Val, Leu, Ile, Thr, Phe, Tyr, Thr et R¹⁰ étant un groupe akyle linéaire ou ramifié ayant de 1 à 6 atomes de carbone;
- W est choisi parmi les acides aminés Val, NVal et Leu;
- X est choisi parmi les acides aminés (D)Ala, (D)Ser, (D)Hiv, (D)Val et (D)Thr; et
- Z est choisi parmi les acides aminés Leu et MeLeu.

9. Pro-drogue selon la revendication 8, **caractérisée en ce que** ladite séquence linéaire de dix acides aminés est choisie parmi les séquences suivantes de formules (III) à (XV):
-Abu-Sar-MeLeu-Val-MeLeu-Ala-(D)Ala-MeLeu-MeLeu-MeVal- (III);
-Abu-(D)MeAla-EtVal-Val-MeLeu-Ala-(D)Ala-MeLeu-MeLeu-MeVal- (IV);
-Thr-Sar-MeLeu-Val-MeLeu-Ala-(D)Ala-MeLeu-MeLeu-MeVal- (V);
-Val-Sar-MeLeu-Val-MeLeu-Ala-(D)Ala-MeLeu-MeLeu-MeVal- (VI);
-NVal-Sar-MeLeu-Val-MeLeu-Ala-(D)Ala-MeLeu-MeLeu-MeVal- (VII);
-Val-(D)MeAla-MeLeu-Val-MeLeu-Ala-(D)Ala-MeLeu-MeLeu-MeVal- (VIII);
-Val-Sar-MeLeu-Val-MeLeu-Ala-(D)Val-MeLeu-Leu-MeVal- (IX);
-Val-Sar-MeLeu-Val-MeLeu-Ala-(D)Thr-MeLeu-Leu-MeVal- (X);
Abu-(D)MeSer(OAc)-MeLeu-Val-MeLeu-Ala-(D)Ala-MeLeu-Leu-MeVal- (XI);
-Abu-Sar-MeLeu-Val-MeLeu-Ala-(D)Ser-MeLeu-MeLeu-MeVal- (XII);
-Thr-Sar-MeLeu-Leu-MeLeu-Ala-(D)Hiv-MeLeu-Leu-MeVal- (XIII);
et
Abu-Sar-MeLeu-Val-MeLeu-Ala-(D)Val-MeLeu-Leu-MeVal- (XIV).

10. Pro-drogue selon l'une quelconque des revendications précédentes ayant respectivement les formules (XV) et (XVI) suivantes: 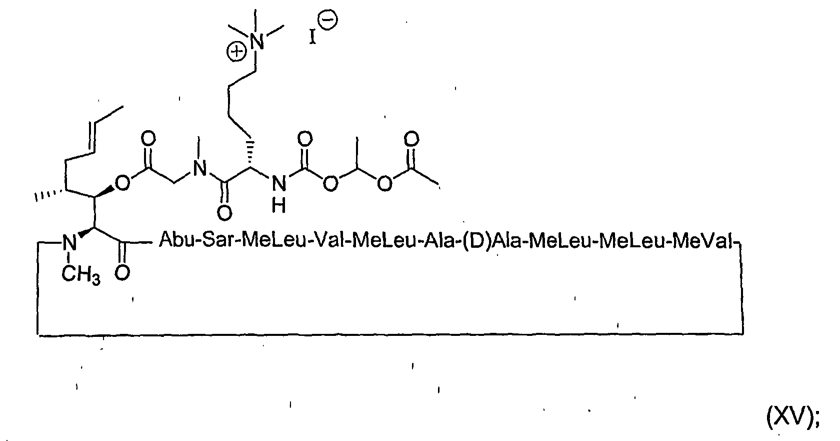 et

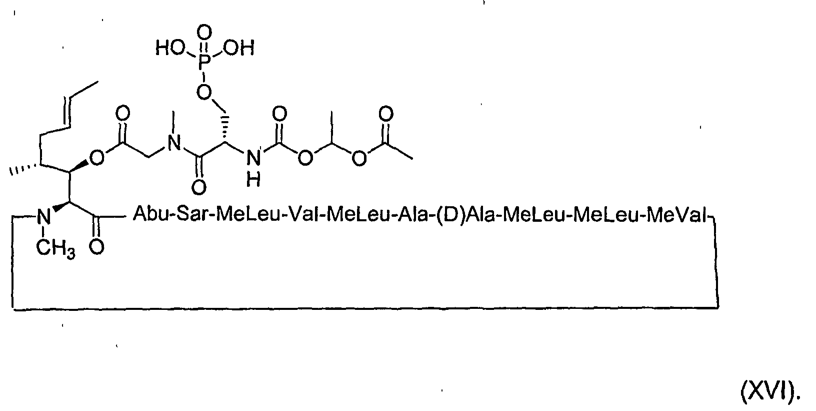

11. Pro-drogue selon la revendication 1 en tant que médicament.

12. Utilisation d'une pro-drogue selon la revendication 1 pour la préparation d'un médicament destiné au traitement de pathologies des muqueuses.

13. Utilisation d'une pro-drogue selon la revendication 1 pour la préparation d'un médicament destiné au traitement local de pathologies oculaires.

14. Utilisation selon la revendication 13, **caractérisée en ce que** lesdites pathologies sont choisies parmi les kératoconjonctivites sèches, le syndrome de Sjögren, les kératoconjonctivites allergiques, en particulier, celles résistantes aux corticostéroïdes, les conjonctivites muco-synéchiantes, les kératites stromales herpétiques, les kératites limbiques immunitaires, la kératite de Thygeson, les pathologies liées aux greffes de cornée, comme traitement adjuvant d'une chirurgie filtrante.

15. Utilisation d'une pro-drogue selon la revendication 1 pour la préparation d'un médicament destiné à permettre la prolongation de survie des allogreffes d'organes.

16. Utilisation selon la revendication 15, **caractérisée en ce que** lesdits organes portant une greffe sont choisis parmi le rein, le coeur, le foie, le pancréas, le poumon, l'intestin grêle, la moelle osseuse.

17. Utilisation d'une pro-drogue selon la revendication 1 pour la préparation d'un médicament destiné à inhiber la réplication du virus de l'immunodéficience humaine de type 1 (VIH-1).

## Patentansprüche

1. Prodrug, bestehend aus einem cyclischen Undecapeptid, das in seiner Peptidkette mindestens einen Aminosäurerest der folgenden allgemeinen Formel (I) aufweist: 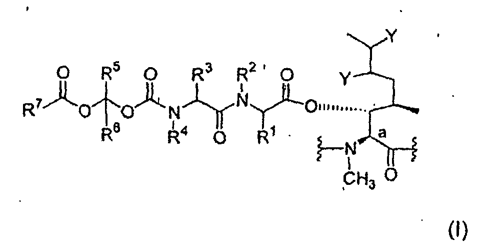 worin:
- das Kohlenstoffatom C^{a} eines der Glieder des Undecapeptidrings darstellt;
- die Substituenten Y jeweils für ein Wasserstoffatom stehen oder gemeinsam eine Bindung bilden;
- die Substituenten R¹ und R³ unabhängig voneinander für ein Wasserstoffatom, eine Aralkylgruppe, eine Alkarylgruppe, eine Heteroalkylgruppe, eine Heterocyclylgruppe, eine Alkylheterocyclylgruppe, eine Heterocyclylalkylgruppe oder eine lineare oder verzweigte Alkylgruppe mit 1 bis 6 Kohlenstoffatomen, gegebenenfalls substituiert durch mindestens eine der unter -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO (OH)(OR⁸), -OPO(OH)₂, -OSO(OR⁸)₂, -OSO(OH)(OR⁸), -OSO(OH)₂ und den verschiedenen versalzten Formen dieser Gruppen ausgewählten Gruppen, wobei jeder der Substituenten R⁸ unabhängig voneinander eine lineare oder verzweigte Alkylgruppe mit 1 bis 6 Kohlenstoffatomen bedeutet, stehen;
- die Substituenten R² und R⁴ unabhängig voneinander für ein Wasserstoffatom, eine Alkarylgruppe oder eine lineare oder verzweigte Alkylgruppe mit 1 bis 6 Kohlenstoffatomen stehen;
- die Substituenten R⁵ und R⁶ unabhängig voneinander für ein Wasserstoffatom, eine Alkarylgruppe oder eine lineare oder verzweigte Alkylgruppe mit 1 bis 6 Kohlenstoffatomen stehen und
- der Substituent R⁷ für eine Aralkylgruppe, eine Alkarylgruppe, eine Heteroalkylgruppe, eine Heterocyclylgruppe, eine Alkylheterocyclylgruppe, eine Heterocyclylalkylgruppe oder eine lineare oder verzweigte Alkylgruppe mit 1 bis 6 Kohlenstoffatomen, wobei diese Gruppen gegebenenfalls durch mindestens eine der unter -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH)₂ und den verschiedenen versalzten Formen dieser Gruppen ausgewählten Gruppen substituiert sind, wobei jeder der Substituenten R⁸ die oben angegebene Bedeutung besitzt, steht.

2. Prodrug nach Anspruch 1, **dadurch gekennzeichnet, daß** in der den Aminosäurerest definierenden allgemeinen Formel (I) mindestens einer der Substituenten R¹ und R³ für eine Aralkylgruppe, eine Alkarylgruppe, eine Heteroalkylgruppe, eine Heterocyclylgruppe, eine Alkylheterocyclylgruppe, eine Heterocyclylalkylgruppe oder eine lineare oder verzweigte Alkylgruppe mit 1 bis 6 Kohlenstoffatomen, wobei jede dieser Gruppen gegebenenfalls durch mindestens eine der unter -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH) ₂ und den verschiedenen versalzten Formen dieser Gruppen ausgewählten Gruppen substituiert ist, wobei jeder der Substituenten R⁸ die oben angegebene Bedeutung besitzt, steht.

3. Prodrug nach Anspruch 2, **dadurch gekennzeichnet, daß** die Aralkyl-, Alkaryl-, Heteroalkyl-, Heterocyclyl-, Alkylheterocyclyl-, Heterocyclylalkyl- oder Alkylgruppen durch mindestens eine der unter -NR⁸₂, -N⁺R⁸₃, -OPO(OH)₂ oder den verschiedenen versalzten Formen dieser Gruppen ausgewählten Gruppen substituiert sind, wobei jeder der Substituenten R⁸ die oben angegebene Bedeutung besitzt.

4. Prodrug nach Anspruch 3, **dadurch gekennzeichnet, daß** mindestens einer der Substituenten R¹ und R³ für eine lineare Alkylgruppe mit 1 bis 6 Kohlenstoffatomen, die durch mindestens eine der unter -NR⁸₂, -N⁺R⁸₃, -OPO(OH)₂ oder den verschiedenen versalzten Formen dieser Gruppen ausgewählten Gruppen substituiert ist, wobei jeder der Substituenten R⁸ die oben angegebene Bedeutung besitzt, steht.

5. Prodrug nach Anspruch 1, **dadurch gekennzeichnet, daß** in der den Aminosäurerest definierenden allgemeinen Formel (I) die Substituenten R⁵ und R⁶ nicht gleichzeitig für ein Wasserstoffatom stehen können.

6. Prodrug nach Anspruch 1, **dadurch gekennzeichnet, daß** in der den Aminosäurerest definierenden allgemeinen Formel (I) mindestens einer der Substituenten R⁵ und R⁶ für eine lineare oder verzweigte Alkylgruppe mit 1 bis 6 Kohlenstoffatomen steht und der Substituent R⁷ für eine Aralkylgruppe oder eine lineare oder verzweigte Alkylgruppe mit 1 bis 6 Kohlenstoffatomen steht.

7. Prodrug nach Anspruch 6, **dadurch gekennzeichnet, daß** die Substituenten R⁵ und R⁶ unabhängig voneinander für ein Wasserstoffatom oder eine Methylgruppe stehen.

8. Prodrug nach Anspruch 1, **dadurch gekennzeichnet, daß** die Peptidkette einen einzigen Aminosäurerest der allgemeinen Formel (I) enthält, der mit einer linearen Sequenz von zehn Aminosäuren der folgenden allgemeinen Formel (II):
-T-U-V-W-MeLeu-Ala-X-MeLeu-Z-MeVal- (II)
worin:
- T unter den Aminosäuren Ala, Abu, Nval, Val und Thr ausgewählt ist;
- U unter den Aminosäuren Sar, (D)MeSer, (D)MeAla und (D) MeSer (OCOR⁹) ausgewählt ist, wobei R⁹ für ein Wasserstoffatom, eine Alkarylgruppe oder eine lineare oder verzweigte Alkylgruppe mit 1 bis 6 Kohlenstoffatomen steht;
- V für eine Aminosäure der allgemeinen Formel (N-R¹⁰) aa steht, wobei aa unter den Aminosäuren Val, Leu, Ile, Thr, Phe, Tyr und Thr ausgewählt ist und R¹⁰ für eine lineare oder verzweigte Alkylgruppe mit 1 bis 6 Kohlenstoffatomen steht;
- W unter den Aminosäuren Val, Nval und Leu ausgewählt ist;
- X unter den Aminosäuren (D)Ala, (D)Ser, (D)Hiv, (D)Val und (D)Thr ausgewählt ist und
- Z unter den Aminosäuren Leu und MeLeu ausgewählt ist;
einen Undecapeptidring bildet.

9. Prodrug nach Anspruch 8, **dadurch gekennzeichnet, daß** die lineare Sequenz von zehn Aminosäuren unter den folgenden Sequenzen der Formeln (III) bis (XV) ausgewählt ist:
-Abu-Sar-MeLeu-Val-MeLeu-Ala-(D)Ala-MeLeu-MeLeu-MeVal- (III);
-Abu-(D)MeAla-EtVal-Val-MeLeu-Ala-(D)Ala-MeLeu-MeLeu-MeVal- (IV);
-Thr-Sar-MeLeu-Val-MeLeu-Ala-(D)Ala-MeLeu-MeLeu-MeVal- (V);
-Val-Sar-MeLeu-Val-MeLeu-Ala-(D)Ala-MeLeu-MeLeu-MeVal- (VI);
-Nval-Sar-MeLeu-Val-MeLeu-Ala-(D)Ala-MeLeu-MeLeu-MeVal- (VII);
-Val-(D)MeAla-MeLeu-Val-MeLeu-Ala-(D)Ala-MeLeu-MeLeu-MeVal- (VIII);
-Val-Sar-MeLeu-Val-MeLeu-Ala-(D)Val-MeLeu-Leu-MeVal- (IX);
-Val-Sar-MeLeu-Val-MeLeu-Ala-(D)Thr-MeLeu-Leu-MeVal- (X);
-Abu-(D)MeSer(OAc)-MeLeu-Val-MeLeu-Ala-(D)Ala-MeLeu-Leu-MeVal- (XI);
-Abu-Sar-MeLeu-Val-MeLeu-Ala-(D)Ser-MeLeu-MeLeu-MeVal- (XII);
-Thr-Sar-MeLeu-Leu-MeLeu-Ala-(D)-Hiv-MeLeu-Leu-MeVal- (XIII)
und
-Abu-Sar-MeLeu-Val-MeLeu-Ala-(D)-Val-MeLeu-Leu-MeVal- (XIV).

10. Prodrug nach einem der vorhergehenden Ansprüche mit den folgenden Formeln (XV) bzw. (XVI): 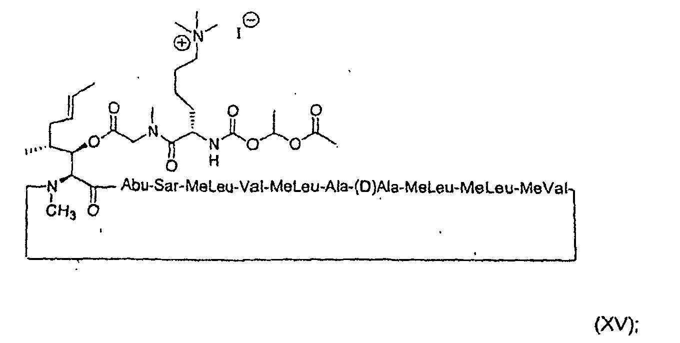 und

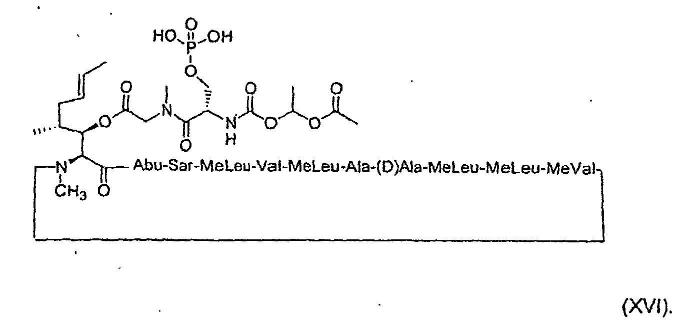

11. Prodrug nach Anspruch 1 zur Verwendung als Arzneimittel.

12. Verwendung eines Prodrugs nach Anspruch 1 zur Herstellung eines Arzneimittels zur Behandlung von pathologischen Zuständen der Schleimhäute.

13. Verwendung eines Prodrugs nach Anspruch 1 zur Herstellung eines Arzneimittels zur Behandlung von pathologischen Zuständen des Auges.

14. Verwendung nach Anspruch 13, **dadurch gekennzeichnet, daß** die pathologischen Zustände unter Keratoconjunctivitis sicca, Sjögren-Syndrom, Keratoconjunctivitis allergica, insbesondere corticosteroidresistenten Formen davon, Conjunctivitis mit Schleimabsonderung und Synechie, stromaler Herpeskeratitis, immunitärer Limbuskeratitis, Thygeson-Keratitis, mit Hornhauttransplantaten verbundenen pathologischen Zuständen und als Hilfsbehandlung bei der filtrierenden Chirurgie ausgewählt sind.

15. Verwendung eines Prodrugs nach Anspruch 1 zur Herstellung eines Arzneimittels zur Verlängerung des Überlebens von Organ-Allotransplantaten.

16. Verwendung nach Anspruch 15, **dadurch gekennzeichnet, daß** die transplantattragenden Organe unter Niere, Herz, Leber, Pankreas, Lunge, Dünndarm und Knochenmark ausgewählt sind.

17. Verwendung eines Prodrugs nach Anspruch 1 zur Herstellung eines Arzneimittels zur Inhibierung der Replikation des HIV-1-Virus.

## Claims

1. Pro-drug consisting of a cyclic undecapeptide in which the peptide chain comprises at least one amino acid residue of general formula (I) below: 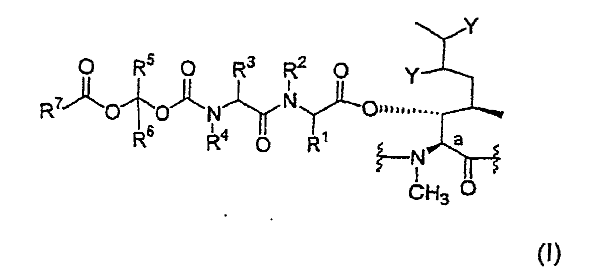 in which:
- the carbon atom C^{a} constitutes one of the links of the undecapeptide ring;
- the substituents Y each represent a hydrogen atom or together constitute a bond;
- the substituents R¹ and R³ represent, independently of one another, a hydrogen atom, an aralkyl group, an alkaryl group, a heteroalkyl group, a heterocyclic group, an alkylheterocyclic group, a heterocyclic alkyl group or a linear or branched alkyl group having from 1 to 6 carbon atoms, optionally substituted with at least one of the groups chosen from -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO (OH)(OR⁸), -OSO(OH)₂, and the various salified forms of these groups, each of the substituents R⁸ representing, independently of one another, a linear or branched alkyl group having from 1 to 6 carbon atoms;
- the substituents R² and R⁴ represent, independently of one another a hydrogen atom, an alkaryl group, or a linear or branched alkyl group having from 1 to 6 carbon atoms;
- the substituents R⁵ and R⁶ represent, independently of one another, a hydrogen atom, an aralkyl group, or a linear or branched alkyl group having from 1 to 6 carbon atoms; and
- the substituent R⁷ represents an aralkyl group, an alkaryl group, a heteroalkyl group, a heterocyclic group, an alkylheterocyclic group, a heterocyclicalkyl group or a linear or branched alkyl group having from 1 to 6 carbon atoms, said groups being optionally substituted with at least one of the groups chosen from -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO(OH)(OR⁸), -OPO(OH)₂, -OSO(OR⁸)₂, -OSO(OH)(OR⁸), -OSO(OH)₂, and the various salified forms of these groups, each of the substituents R⁸ having the definition above.

2. Pro-drug according to Claim 1, **characterized in that**, in general formula (I) defining said amino acid residue, at least one of the substituents R¹ and R³ represents an aralkyl group, an alkaryl group, a heteroalkyl group, a heterocyclic group, an alkylheterocyclic group, a heterocyclic alkyl group or a linear or branched alkyl group having from 1 to 6 carbon atoms, each of said groups being substituted with at least one of the groups chosen from -COOH, -CONHR⁸, -NHC=NH(NH₂), -NHC=NR⁸(NH₂), -NH₂, -NHR⁸, -NR⁸₂, -N⁺R⁸₃, -OH, -OPO(OR⁸)₂, -OPO (OH)(OR⁸), -OPO(OH)₂, -OSO(OR⁸)_{2,} -OSO(OH)(OR⁸), -OSO(OH)₂, and the various salified forms of these groups, each of the substituents R⁸ having the definition above.

3. Pro-drug according to Claim 2, **characterized in that** said aralkyl, alkaryl, heteroalkyl, heterocyclic, alkylheterocyclic, heterocyclicalkyl or alkyl groups are substituted with at least one of the groups chosen from -NR⁸₂, -N⁺R⁸₃, -OPO (OH) ₂ or the various salified forms of these groups, each of the substituents R⁸ having the definition above.

4. Pro-drug according to Claim 3, **characterized in that** at least one of said substituents R¹ and R³ represents a linear alkyl group having from 1 to 6 carbon atoms substituted with at least one of the groups chosen from -NR⁸₂, -N⁺R⁸₃, -OPO(OH)₂ or the various salified forms of these groups, each of the substituents R⁸ having the definition above.

5. Pro-drug according to Claim 1, **characterized in that**, in general formula (I) defining said amino acid residue, the substituents R⁵ and R⁶ cannot simultaneously represent a hydrogen atom.

6. Pro-drug according to Claim 1, **characterized in that**, in general formula (I) defining said amino acid residue, at least one of said substituents R⁵ and R⁶ represents a linear or branched alkyl group having from 1 to 6 carbon atoms, and the substituent R⁷ represents an aralkyl group or a linear or branched alkyl group having from 1 to 6 carbon atoms.

7. Pro-drug according to Claim 6, **characterized in that** said substituents R⁵ and R⁶ represent, independently of one another, a hydrogen atom or a methyl group.

8. Pro-drug according to Claim 1, **characterized in that** said peptide chain comprises a single amino acid residue of general formula (I) forming an undecapeptide ring with a linear sequence of ten amino acids of general formula (II) below:
-T-U-V-W-MeLeu-Ala-X-MeLeu-Z-MeVal- (II)
in which:
- T is chosen from the amino acids Ala, Abu, Nval, Val and Thr;
- U is chosen from the amino acids Sar, (D)MeSer, (D)MeAla and (D)MeSer(OCOR⁹), with R⁹ representing a hydrogen atom, an alkaryl group, or a linear or branched alkyl group having from 1 to 6 carbon atoms;
- V represents an amino acid of general formula (N-R¹⁰) aa, aa being chosen from the amino acids Val, Leu, Ile, Thr, Phe, Tyr and Thr, and R¹⁰ being a linear or branched alkyl group having from 1 to 6 carbon atoms;
- W is chosen from the amino acids Val, Nval and Leu;
- X is chosen from the amino acids (D)Ala, (D)Ser, (D)Hiv, (D)Val and (D)Thr; and
- Z is chosen from the amino acids Leu and MeLeu.

9. Pro-drug according to Claim 8, **characterized in that** said linear sequence of ten amino acids is chosen from the following sequences of formulae (III) to (XV) :
-Abu-Sar-MeLeu-Val-MeLeu-Ala-(D)Ala-MeLeu-MeLeu-MeVal- (III);
-Abu-(D)MeAla-EtVal-Val-MeLeu-Ala-(D)Ala-MeLeu-MeLeu-MeVal- (IV);
-Thr-Sar-MeLeu-Val-MeLeu-Ala-(D)Ala-MeLeu-MeLeu-MeVal- (V);
-Val-Sar-MeLeu-Val-MeLeu-Ala-(D)Ala-MeLeu-MeLeu-MeVal- (VI);
-Nval-Sar-MeLeu-Val-MeLeu-Ala-(D)Ala-MeLeu-MeLeu-MeVal- (VII);
-Val-(D)MeAla-MeLeu-Val-MeLeu-Ala-(D)Ala-MeLeu-MeLeu-MeVal- (VIII);
-Val-Sar-MeLeu-Val-MeLeu-Ala-(D)Val-MeLeu-Leu-MeVal- (IX);
-Val-Sar-MeLeu-Val-MeLeu-Ala-(D)Thr-MeLeu-Leu-MeVal- (X);
-Abu-(D)MeSer(OAc)-MeLeu-Val-MeLeu-Ala-(D)Ala-MeLeu-Leu-MeVal- (XI);
-Abu-Sar-MeLeu-Val-MeLeu-Ala-(D)Ser-MeLeu-MeLeu-MeVal- (XII);
-Thr-Sar-MeLeu-Leu-MeLeu-Ala-(D)-Hiv-MeLeu-Leu-MeVal- (XIII);
and
-Abu-Sar-MeLeu-Val-MeLeu-Ala-(D)-Val-MeLeu-Leu-MeVal- (XIV).

10. Pro-drug according to any one of the preceding claims, having, respectively, formulae (XV) and (XVI) below: 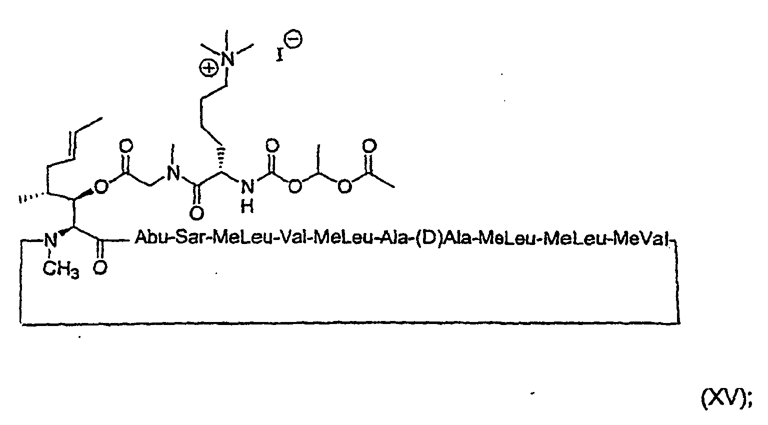 and

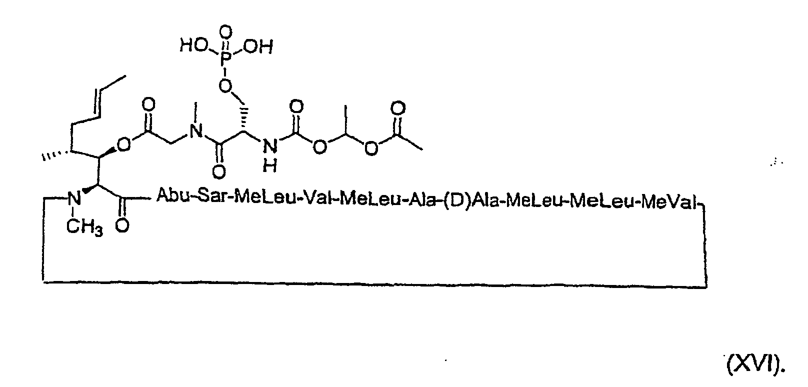

11. Pro-drug according to Claim 1, for use as a medicinal product.

12. Use of a pro-drug according to Claim 1, for preparing a medicinal product intended for the treatment of pathological conditions of the mucous membranes.

13. Use of a pro-drug according to Claim 1, for preparing a medicinal product intended for the local treatment of pathological conditions of the eye.

14. Use according to Claim 13, **characterized in that** said pathological conditions are chosen from dry keratoconjunctivitis, Sjögren's syndrome, forms of allergic keratoconjunctivitis, in particular those resistant to corticosteroids, conjunctivitis producing mucous and synechia, herpetic stromal keratitis, immune-related limbic keratitis and Thygeson's keratitis, and prevention of corneal transplant rejection, and as an adjuvant treatment for filtering surgery.

15. Use of a pro-drug according to Claim 1, for preparing a medicinal product intended to allow prolonged survival of organ allografts.

16. Use according to Claim 15, **characterized in that** said organs bearing a graft are chosen from the kidney, heart, liver, pancreas, lung, small intestine and bone marrow.

17. Use of a pro-drug according to Claim 1, for preparing a medicinal product intended to inhibit replication of the human immunodeficiency virus type I (HIV-1).
